# EUROPEAN PATENT APPLICATION

(11) **EP 1 604 661 A1**
(43) Date of publication of application: **14.12.2005**
(21) Application number: 04715512.2
(22) Date of filing: 27.02.2004
(51) Int. Cl.: A61K 31/365, C07D 493/22, A61P 27/02

(54) **REMEDY FOR CORNEAL ULCER**

(30) Priority: 27.02.2003 JP 2003052072
(71) Applicant: Nishida, Teruo, Ube-shi, Yamaguchi 755-0152 (JP); Senju Pharmaceutical Co., Ltd., Osaka-shi, Osaka 541-0046 (JP)
(72) Inventor: NISHIDA, Teruo, 7550152 (JP); NAKAMURA, Yoshikuni, Nishi-ku, Kobe-shi,Hyogo 651-2241 (JP)
(74) Representative: von Kreisler, Alek
(86) International application number: PCT/JP2004/002406
(87) International publication number: WO 2004/075888

(57) **Abstract**

The present invention provides a pharmaceutical agent capable of effectively treating corneal ulcer, more particularly, a therapeutic agent for corneal ulcer, which contains triptolide or a derivative thereof or a pharmaceutically acceptable salt thereof.

## Description

### Technical Field

The present invention relates to a therapeutic agent for corneal ulcer. More particularly, the present invention relates to a therapeutic agent for corneal ulcer, which contains triptolide or a derivative thereof.

### Background Art

Cornea consists of epithelial cells, Bowman's membrane, corneal stroma, Descemet's membrane and endothelial cells. Cornea is normally avascular tissue, and receives nutrients from the aqueous humor in the anterior chamber of eye instead of blood vessels. Cornea does not have immunocytes, either. Therefore, cornea is a very special tissue as compared to other tissues.

Corneal ulcer refers to the condition where corneal stroma (hereinafter sometimes to be simply referred to as stroma) mainly consisting of collagen is lysed and deleted by the activation and hypersecretion of collagenolytic enzyme. As the collagenolytic enzyme causing corneal ulcer, bacterial collagenase and matrix metalloproteases (MMPs) are known. When cornea is infected with bacteria and the like, bacterial collagenase is secreted, which directly degrades collagen in corneal stroma and causes corneal ulcer. Simultaneously, microorganisms such as bacteria and the like secrete other enzymes and toxins, and these factors become biological signals causing activation of corneal stromal cells (sometimes referred to as corneal fibroblasts). The activated corneal stromal cells produce inactive MMPs (proMMPs) and secrete proMMPs. Moreover, proMMPs become active MMPs due to the enhanced activation mechanism and limited proteolysis by bacterial enzymes, and the active MMPs cause corneal ulcer (control of the activity of activated MMPs depends on the concentration of TIMPs (Tissue Inhibitors of Metalloproteinases)). The changes in the extracellular environment caused by the degradation of stromal collagen promote ulcer by the production and activation of MMPs in association with the activation of corneal stromal cells, or MMPs secreted by the infiltrated inflammatory cells, thus producing a vicious circle of activation of corneal stromal cells and degradation of corneal stroma. When the bacteria are killed by antibiotic and the like, secretion of bacterial collagenase is suppressed and direct corneal stroma degradation due to the bacteria is suppressed. However, since antibiotic and the like cannot suppress activation of corneal stromal cell caused by the biological signals once transmitted from bacteria and the like to corneal stromal cells, progression of ulcer is clinically observed from time to time. The factor to suppress such a vicious circle is considered to be only steroids (NISHIDA Teruo et al. ed., "Monthly ophthalmology therapeutic practice (vol. 79) pharmacotherapeutics of corneal and conjunctival diseases", 1st printing, Bunkodo, January 2002, p. 9-11).

Triptolide is a main active ingredient of Tripterygium wilfordii. Triptolide and derivatives thereof are known to have an immunosuppressive action, and use thereof as a therapeutic agent for autoimmune diseases and anti-inflammatory agents (e.g., WO97/31920, JP-A-2001-504437, WO96/08262, WO98/52951, WO00/12483, WO02/28862, WO00/63212, WO98/52933, US Patent No. 5972998, US Patent No. 6004999, WO02/070472, and WO02/074759). However, it has not been described or suggested that triptolide and derivatives thereof show suppression of proMMPs production and suppression of proMMPs activation in the cornea.

Fini et al. describe in "Archives of Dermatological Research", Germany, 1998, vol. 290 Suppl., p. S12-S23, that a synthetic MMP inhibitor inhibits progression of corneal ulcer in animal model by inhibition of corneal MMPs. However, suppression of proMMPs production and suppression of proMMPs activation in cornea by triptolide and derivatives thereof are not described or suggested.

Lin et al. describe in "ARTHRITIS & RHEUMATISM", (U.S.A.), September 2001, vol. 44, No. 9, p. 2193-2220 that triptolide suppresses production of proMMPs 1 and 3, which are MMPs precursors, in human synovial fluid fibroblast. However, there is no description suggesting that triptolide and derivative thereof suppress proMMPs production and proMMPs activation in cornea as well.

### Disclosure of the Invention

As mentioned above, steroid is currently a sole therapeutic agent for corneal ulcer that can suppress a vicious circle of activation of corneal stromal cell and corneal stroma degradation. However, steroid is known to cause various side effects, and use of steroid in the treatment of corneal ulcer often aggravates the condition. Thus, the present inventors have conducted intensive studies in an attempt to create a pharmaceutical agent capable of breaking the above-mentioned vicious circle and treating corneal ulcer even without using steroid, and found that triptolide and derivatives thereof effectively inhibit production and activation of proMMPs in cornea without affecting the production of TIMPs, whereby the symptoms of corneal ulcer can be treated or remitted, which resulted in the completion of the present invention.

Accordingly, the present invention provides the following.
(1) A therapeutic agent for corneal ulcer, which comprises triptolide or a derivative thereof or a pharmaceutically acceptable salt thereof.
(2) The therapeutic agent of the above-mentioned (1), which is used for topical administration to the eye.
(3) The therapeutic agent of the above-mentioned (2), which is an ophthalmic solution.
(4) A pharmaceutical composition for the treatment of corneal ulcer, which comprises triptolide or a derivative thereof or a pharmaceutically acceptable salt thereof.
(5) The pharmaceutical composition of the above-mentioned (4), which is used for topical administration to the eye.
(6) The pharmaceutical composition of the above-mentioned (5), which is used for an ophthalmic solution.
(7) A method for treating corneal ulcer, which comprises administering triptolide or a derivative thereof or a pharmaceutically acceptable salt thereof.
(8) The treatment method of the above-mentioned (7), wherein the administration is a topical administration to the eye.
(9) The treatment method of the above-mentioned (8), wherein the topical administration is that of an ophthalmic solution.
(10) Use of triptolide or a derivative thereof or a pharmaceutically acceptable salt thereof, for the production of a therapeutic agent for corneal ulcer.
(11) The use of the above-mentioned (10), wherein the therapeutic agent for corneal ulcer is used for topical administration to the eye.
(12) The use of the above-mentioned (11), wherein the therapeutic agent for corneal ulcer is an ophthalmic solution.
(13) A commercial package comprising the therapeutic agent of any of the above-mentioned (1)-(3) and a written matter associated therewith, the written matter stating that the therapeutic agent can or should be used for treating corneal ulcer.
(14) A commercial package comprising the pharmaceutical composition of any of the above-mentioned (4)-(6) and a written matter associated therewith, the written matter stating that the pharmaceutical composition can or should be used for treating corneal ulcer.

### Brief Description of the Drawings

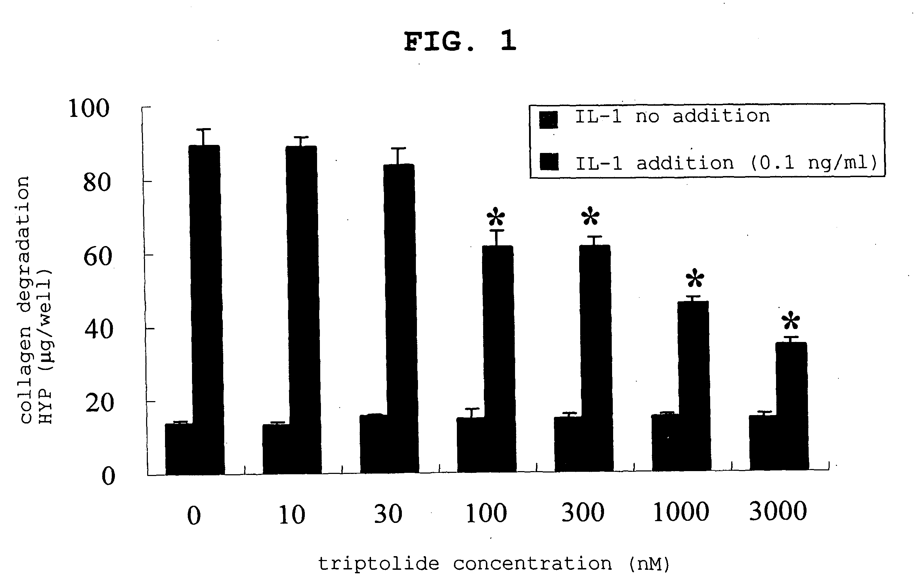

Fig. 1 is a graph showing the effect of triptolide on the collagen degradation induced by IL-1β, in terms of the amount of degraded collagen expressed in a hydroxyproline amount per 1 well (N=3, mean±SEM, IL-1β concentration 0.1 ng/ml, *p<0.001 (Sheffe method)).

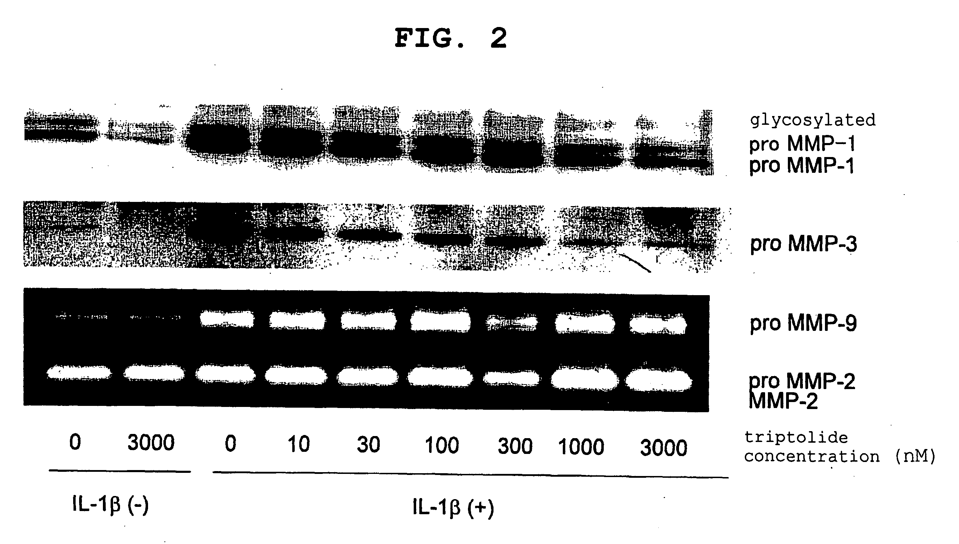

Fig. 2 shows the effect of triptolide on the expression of MMP and proMMPs in rabbit corneal stromal cells. The cells were cultured under plasminogen non-addition conditions, and the results of Western blot performed for glycosylated proMMP-1, proMMP-1 and proMMP-3 and gelatin zymography performed for proMMP-9, proMMP-2 and MMP-2 are shown. IL-1β(-) shows the results of IL-1β non-addition and IL-1β(+) shows the results of IL-1β addition.

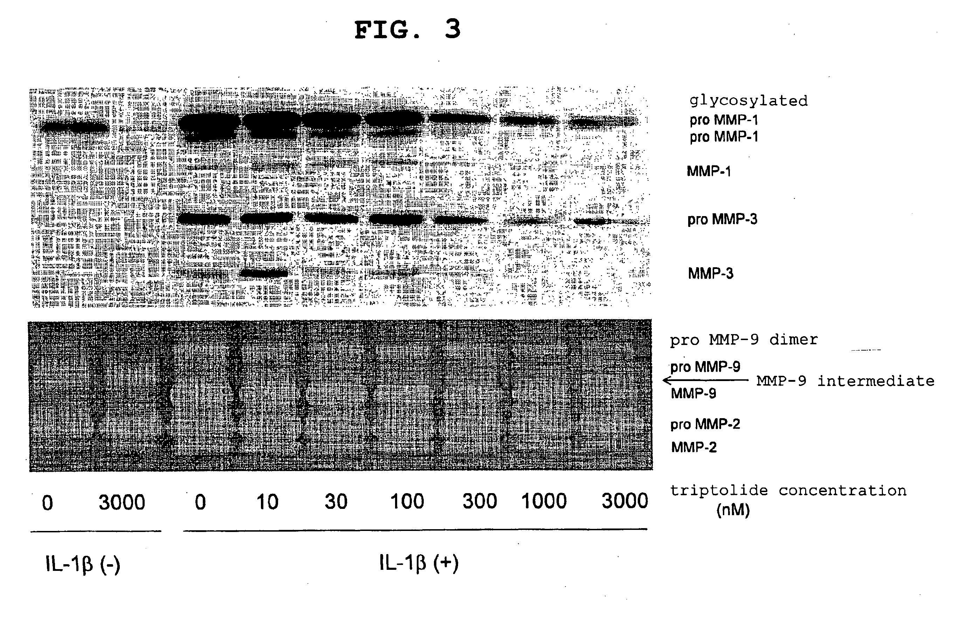

Fig. 3 shows the effect of triptolide on the expression of MMPs and proMMPs in rabbit corneal stromal cells. The cells were cultured under the conditions of 60 mg/ml of plasminogen addition, and the results of Western blot performed for glycosylated proMMP-1, proMMP-1, MMP-1, proMMP-3 and MMP-3 and gelatin zymography performed for proMMP-9 dimer, proMMP-9, MMP-9 intermediate, MMP-9, proMMP-2 and MMP-2 are shown. IL-1β(-) shows the results of IL-1β non-addition and IL-1β(+) shows the results of IL-1β addition.

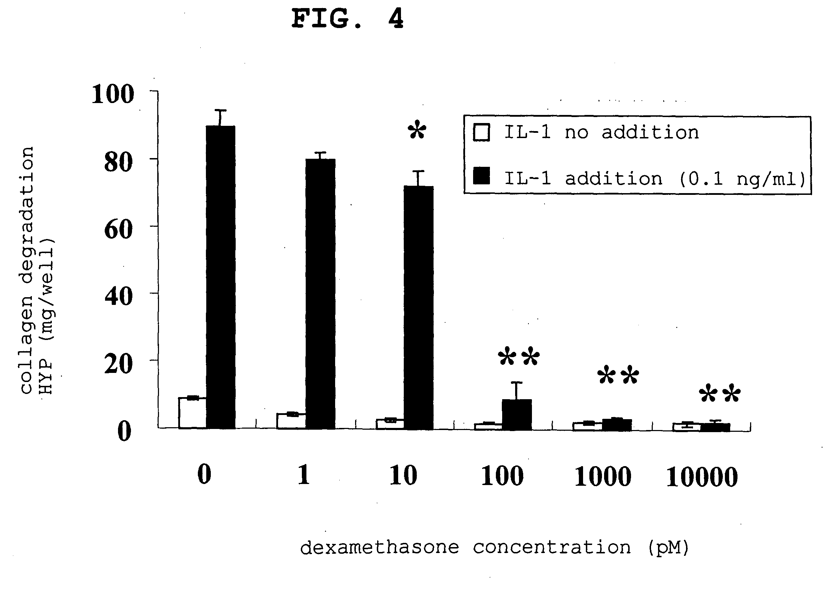

Fig. 4 is a graph showing the effect of dexamethasone on the collagen degradation induced by IL-1β, in terms of the amount of degraded collagen expressed in a hydroxyproline amount per 1 well (N=3, mean±SEM, IL-1β concentration 0.1 ng/ml, *p<0.05, **p<0.001 (Sheffe method)).

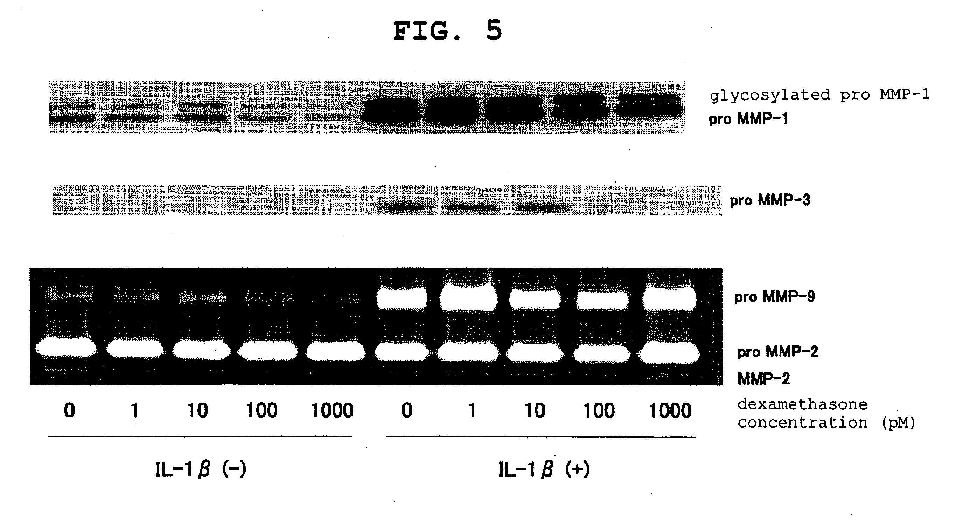

Fig. 5 shows the effect of dexamethasone on the expression of MMP and proMMPs in rabbit corneal stromal cells. The cells were cultured under plasminogen non-addition conditions, and the results of Western blot performed for glycosylated proMMP-1, proMMP-1 and proMMP-3 and gelatin zymography performed for proMMP-9, proMMP-2 and MMP-2 are shown. IL-1β(-) shows the results of IL-1β non-addition and IL-1β(+) shows the results of IL-1β addition.

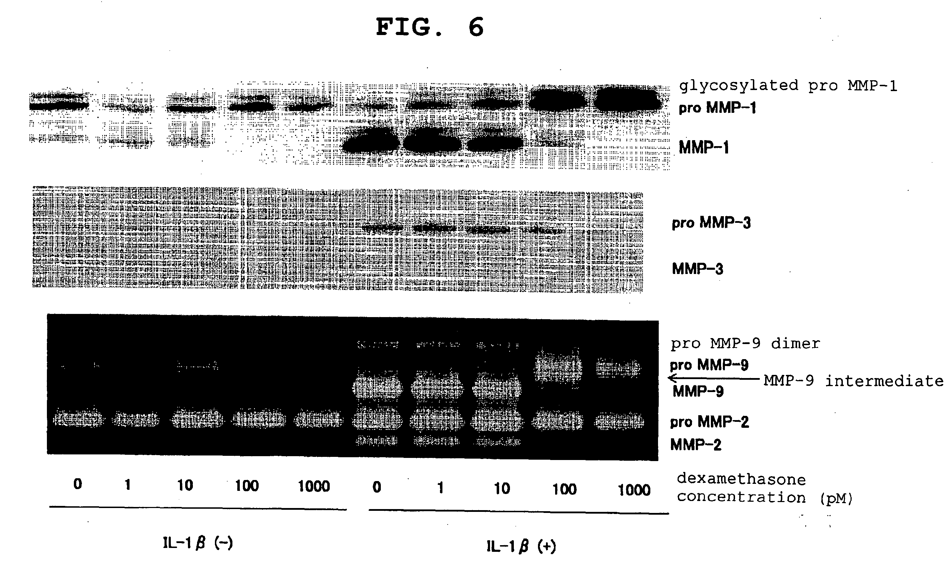

Fig. 6 shows the effect of dexamethasone on the expression of MMPs and proMMPs in rabbit corneal stromal cells. The cells were cultured under the conditions of 60 mg/ml of plasminogen addition, and the results of Western blot performed for glycosylated proMMP-1, proMMP-1, MMP-1, proMMP-3 and MMP-3 and gelatin zymography performed for proMMP-9 dimer, proMMP-9, MMP-9 intermediate, MMP-9, proMMP-2 and MMP-2 are shown. IL-1β(-) shows the results of IL-1β non-addition and IL-1β(+) shows the results of IL-1β addition.

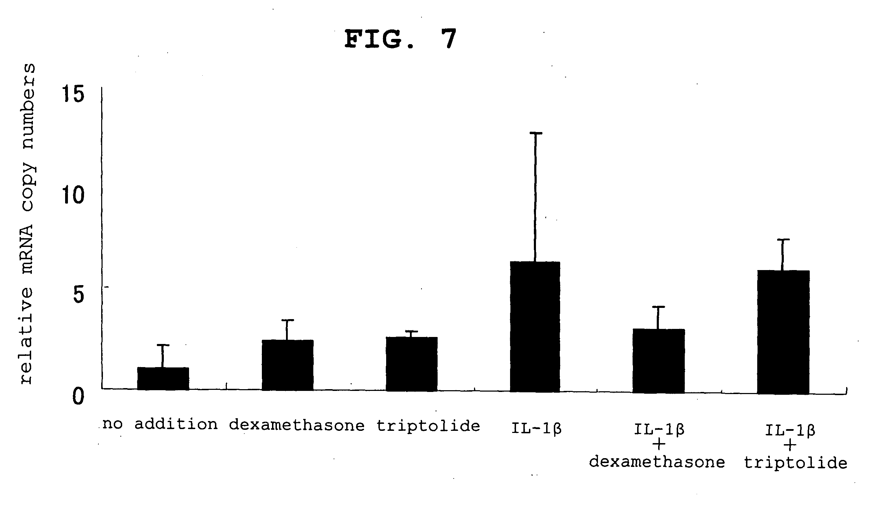

Fig. 7 is a graph showing TIMP-1 production in terms of relative value of the copy number of mRNA (N=2-4, mean±SD, IL-1β concentration 0.1 ng/ml, dexamethasone concentration 1 nM, triptolide concentration 3 µM).

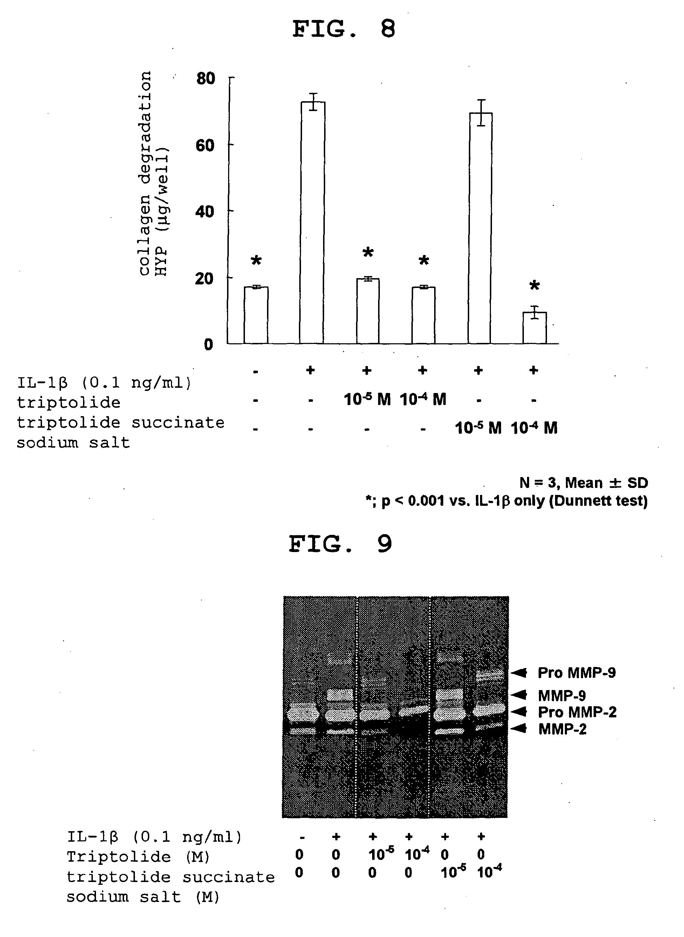

Fig. 8 is a graph showing the effect of triptolide and triptolide succinate sodium salt on the collagen degradation induced by IL-1β, in terms of the amount of degraded collagen expressed in a hydroxyproline amount per 1 well. IL-1β(-) shows the results of IL-1β non-addition and IL-1β(+) shows the results of IL-1β addition (N=3, mean±SD, IL-1β concentration 0.1 ng/ml, *p<0.001 (Dunnet method)).
Fig. 9 shows the effect of triptolide and triptolide succinate sodium salt on the expression of MMPs and proMMPs by rabbit corneal stromal cells. The cells were cultured under plasminogen addition conditions, and the results of gelatin zymography performed for proMMP-9, MMP-9, proMMP-2 and MMP-2 are shown. IL-1β(-) shows the results of IL-1β non-addition and IL-1β(+) shows the results of IL-1β addition. IL-1β concentration 0.1 ng/ml.

### Detailed Description of the Invention

The corneal ulcer in the present description refers to the state where corneal epithelium is defective, and stroma of the defective epithelium is partially deleted, which may be derived from biological stimulation such as bacterial infection and the like or chemical or physical stimulation, and the causal factor is not particularly limited.

The triptolide in the present Description refers to a compound having the following structure, which may be naturally occurring or synthesized.

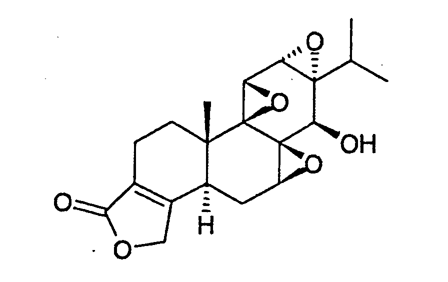

As the naturally occurring triptolide, those derived from *Tripterygium wilfordii* can be mentioned. For example, a solvent extract such as an ethanol extract of *Tripterygium wilfordii* as described in WO98/52951, such extract after further purification and the like can be mentioned. Any final form such as a liquid, a solid etc. can be used for the present invention as long as it is derived from *Tripterygium wilfordii.*

As the derivative triptolide in the present Description, triptolide derivatives described in WO97/31920, JP-A-2001-504437, WO96/08262, WO98/52951, WO00/12483, WO02/28862, WO00/63212, WO98/52933, US Patent No. 5972998, US Patent No. 6004999, WO02/070472 and WO02/074759 can be mentioned. These derivatives can be synthesized by referring to each publication. The triptolide derivatives described in respective publications are concretely described in the following.

WO97/31920 and JP-A-2001-504437 describe triptolide derivatives such as
triptolide succinate 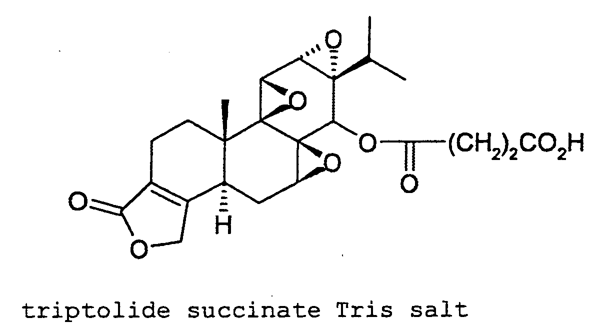 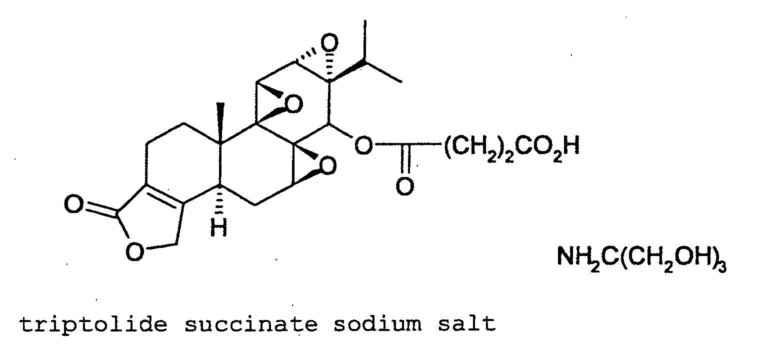 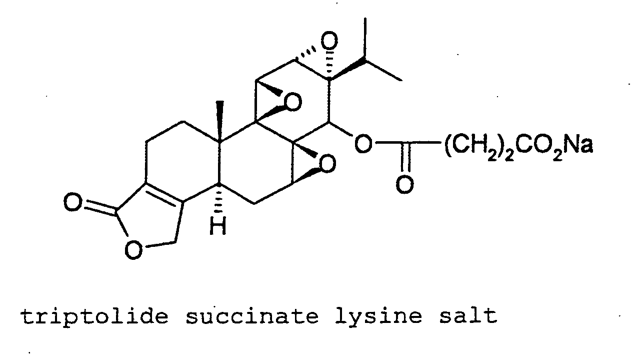 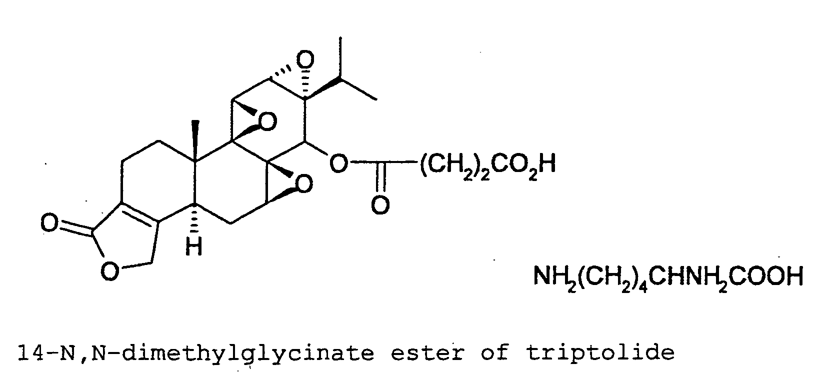 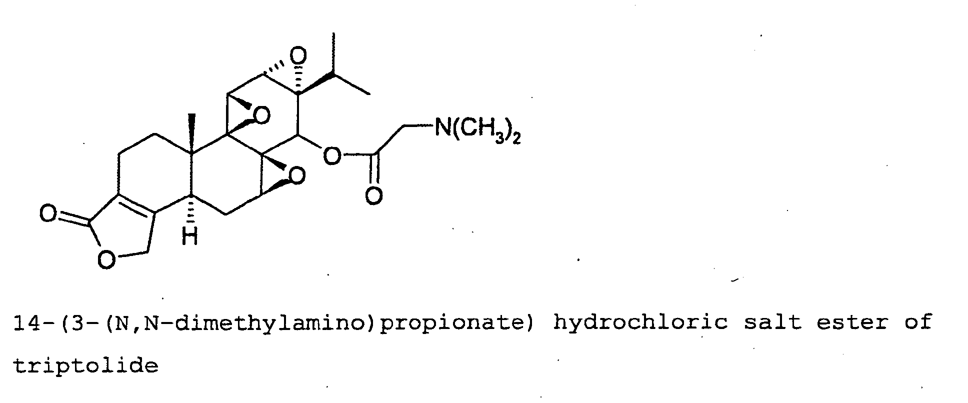 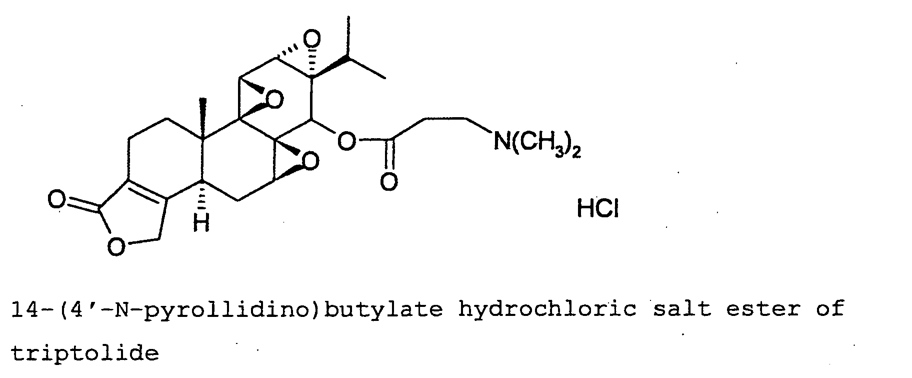 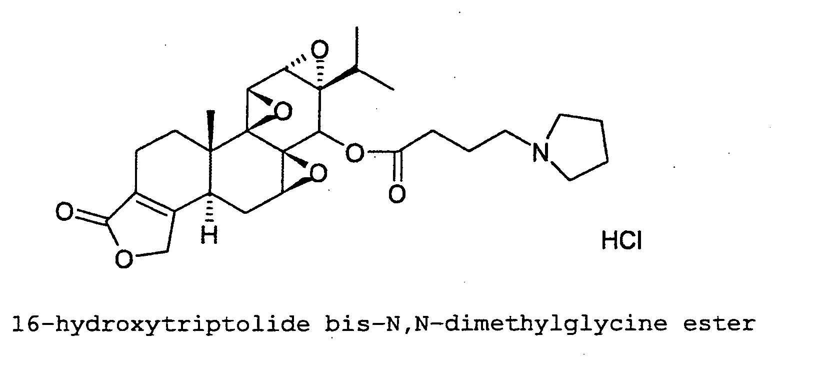 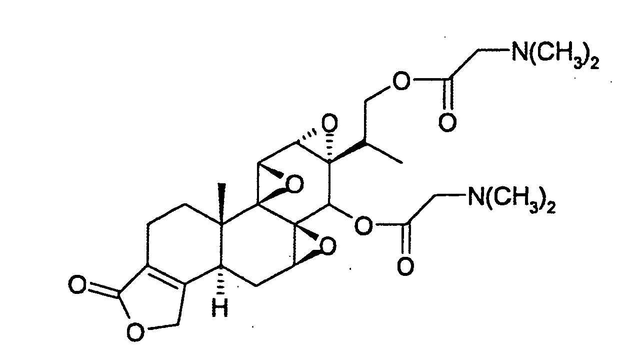 and the like.

WO96/08262 describes an ethanol extract of *Tripterygium wilfordii* having the structure shown below and a purified triptolide component thereof.

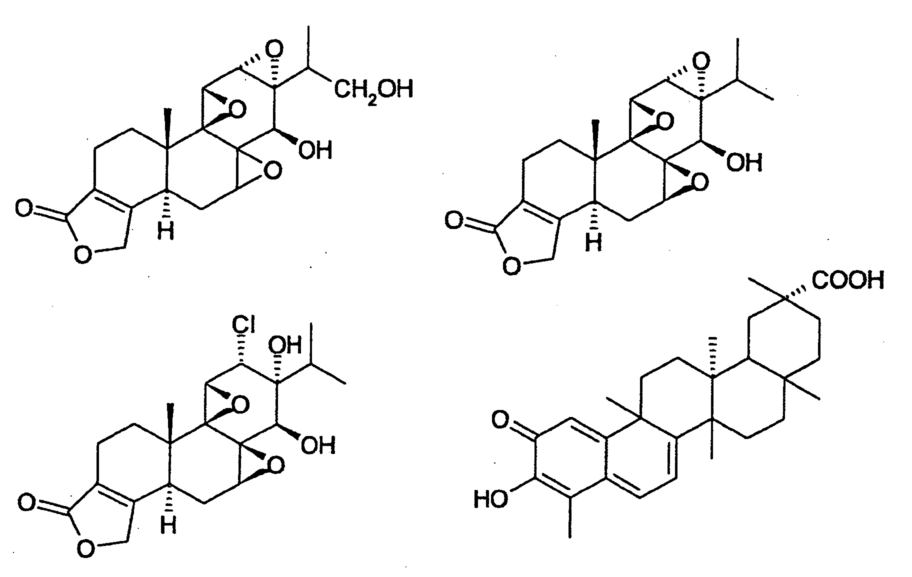

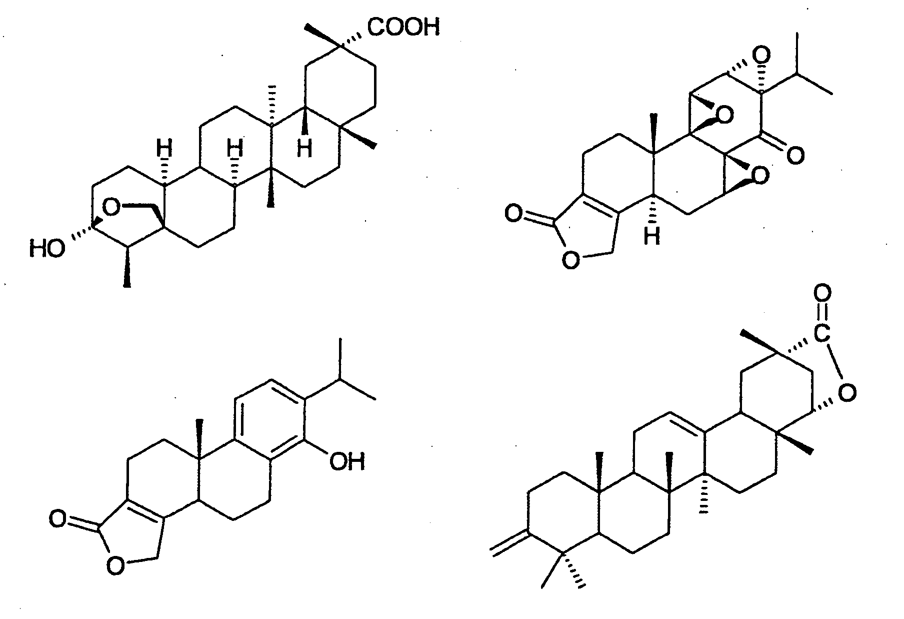

WO98/52951 and US Patent No. 6004999 describe triptolide derivatives such as
[5aR-(5aα,6α,6aα,7aα,7bβ,8aS*,8bα)]-3,3b,4,5,5a,6,6a,7a,7b,8b,9,10-dodecahydro-5a,6-dihydroxy-8b-methyl-6a-(1-methylethyl)-1H-bisoxireno[4b,5:6,7]phenanthro[1,2-c]furan-1-one 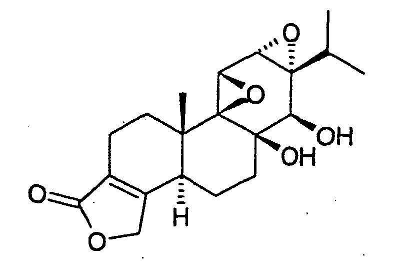
[1S-(1α,2β,3α,3aβ,4aR*,4bα,11aα)]-3-chloro-1,2,3,3a,4b,5,6,9,9b,10,11,11a-dodecahydro-1,2,11a-trihydroxy-4b-methyl-2-(1-methylethyl)-7H-oxireno[4b,5]phenanthro[1,2-c]furan-7-one 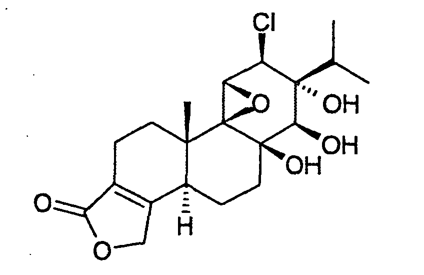
[1S-(1α,2β,3α,3aβ,4aR*,4bα,11β,11aα)]-3-chloro-1,2,3,3a,4b,5,6,9,9b,10,11,11a-dodecahydro-1,11a-dihydroxy-4b-methyl-2-(1-methylethyl)-2,11-epoxy-7H-oxireno[4b,5]phenanthro[1,2-c]furan-7-one 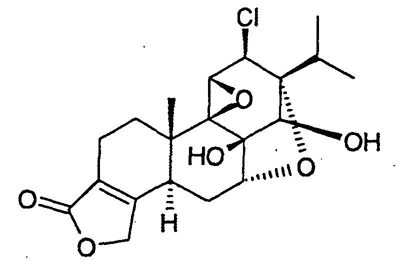
[1S-(1α,2β,3α,3aβ,4aR*,4bα,11β,11aα)]-3-bromo-1,2,3,3a,4b,5,6,9,9b,10,11,11a-dodecahydro-1,11a-dihydroxy-4b-methyl-2-(1-methylethyl)-2,11-epoxy-7H-oxireno[4b,5]phenanthro[1,2-c]furan-7-one 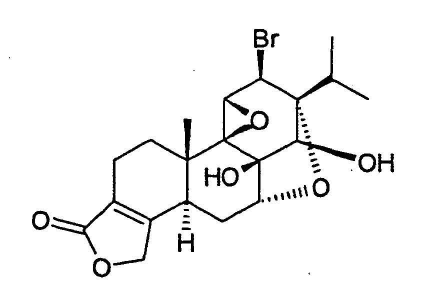
[3bS-(3bα,5aβ,6β,6aβ,7aβ,7bα,8aR*,8bβ,10β)]-3,3b,4,5,5a,6,6a,7a,7b,8b,9,10-dodecahydro-5a,6,10-trihydroxy-8b-methyl-6a-(1-methylethyl)-1H-bisoxireno[4b,5:6,7]phenanthro[1,2-c]furan-1-one 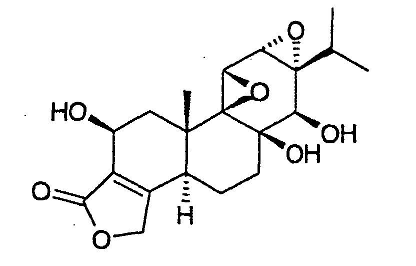
[1S-(1α,2β,3α,3aβ,4aR*,4bα,6α,9bβ,11aα)]-3-chloro-1,2,3,3a,4b,5,6,9,9b,10,11,11a-dodecahydro-1,2,6,11a-tetrahydroxy-4b-methyl-2-(1-methylethyl)-7H-oxireno[4b,5]phenanthro[1,2-c]furan-7-one 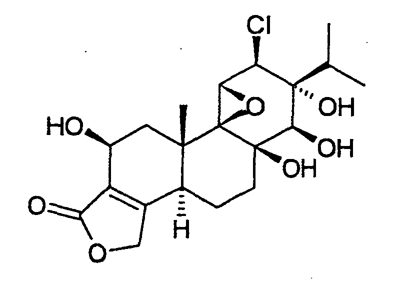
[1S-(1α,2β,3α,3aβ,4aR*,4bα,6α,9bβ,11β,11aα)]-3-chloro-1,2,3,3a,4b,5,6,9,9b,10,11,11a-dodecahydro-1,6,11a-trihydroxy-4b-methyl-2-(1-methylethyl)-2,11-epoxy-7H-oxireno[4b,5]phenanthro[1,2-c]furan-7-one 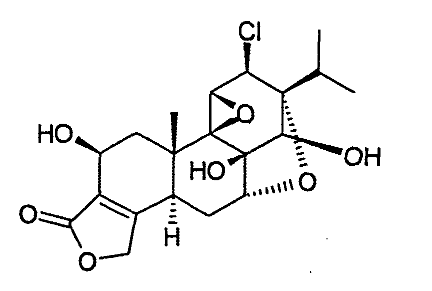
[3bS-(3bα,5aβ,6β,6aβ,7aβ,7bα,8aR*,8bβ)]-3,3b,4,5,5a,6,6a,7a,7b,8b,9,10-dodecahydro-5a,6-dihydroxy-6a-(1-hydroxy-1-methylethyl)-8b-methyl-1H-bisoxireno[4b,5:6,7]phenanthro[1,2-c]furan-1-one 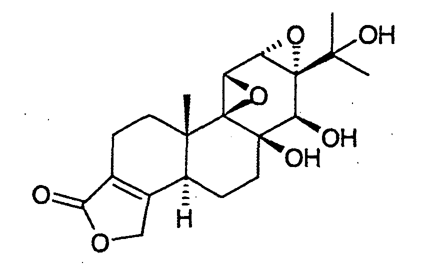
[1S-(1α,2β,3α,3aβ,4aR*,4bα,9bβ,11aα)]-3-chloro-1,2,3,3a,4b,5,6,9,9b,10,11,11a-dodecahydro-1,2,11a-trihydroxy-2-(1-hydroxy-1-methylethyl)-4b-methyl-7H-oxireno[4b,5]phenanthro[1,2-c]furan-7-one 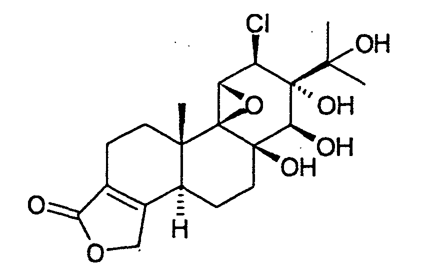
[1S-(1α,2β,3α,3aβ,4aR*,4bα,9bβ,11β,11aα)]-3-chloro-1,2,3,3a,4b,5,6,9,9b,10,11,11a-dodecahydro-1,11a-dihydroxy-2-(1-hydroxy-1-methylethyl)-4b-methyl-2,11-epoxy-7H-oxireno[4b,5]phenanthro[1,2-c]furan-7-one 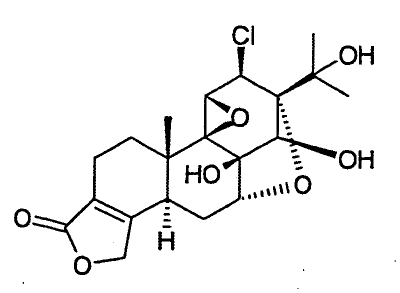
[3bS-(3bα,5aβ,6β,6aβ,7aβ,7bα,8aR*,8bβ)]-6a-(2-hydroxy-1-methylethyl)-3,3b,4,5,5a,6,6a,7a,7b,8b,9,10-dodecahydro-5a,6-dihydroxy-8b-methyl-1H-bisoxireno[4b,5:6,7]phenanthro[1,2-c]furan-1-one 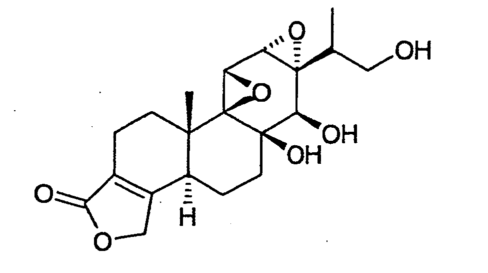
[1S-(1α,2β,3α,3aβ,4aR*,4bα,9bβ,11aα)]-3-chloro-2-(2-hydroxy-1-methylethyl)-1,2,3,3a,4b,5,6,9,9b,10,11,11a-dodecahydro-1,2,11a-trihydroxy-4b-methyl-7H-oxireno[4b,5]phenanthro[1,2-c]furan-7-one 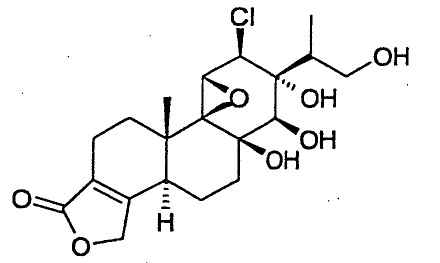
[1S-(1α,2β,3α,3aβ,4aR*,4bα,9bβ,11β,11aα)]-3-chloro-2-(2-hydroxy-1-methylethyl)-1,2,3,3a,4b,5,6,9,9b,10,11,11a-dodecahydro-1,11a-dihydroxy-4b-methyl-1,11-epoxy-7H-oxireno[4b,5]phenanthro[1,2-c]furan-7-one 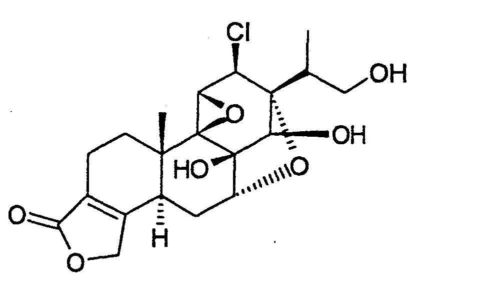
[3bS-(3bα,5aβ,6β,6aβ,7aβ,7bα,8aR*,8bβ)]-mono[3,3b,4,5,5a,6,6a,7a,7b,8b,9,10-dodecahydro-5a-hydroxy-8b-methyl-6a-(1-methylethyl)-1-oxo-1H-bisoxireno[4b,5:6,7]phenanthro[1,2-c]furan-6-yl]ester, butanedioic acid 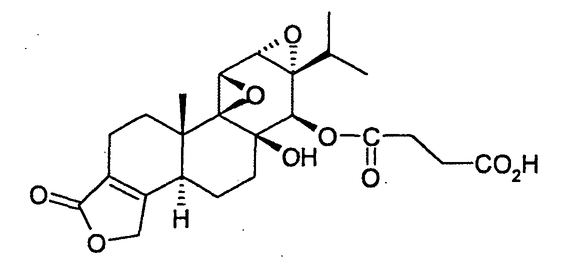
[1S-(1α,2β,3α,3aβ,4aR*,4bα,9bβ,11aα)]-mono[3-chloro-1,3,3a,4b,5,6,7,9,9b,10,11,11a-dodecahydro-2,11a-dihydroxy-4b-methyl-2-(1-methylethyl)-7-oxo-2H-oxireno[4b,5]phenanthro[2,1-c]furan-1-yl]ester, butanedioic acid 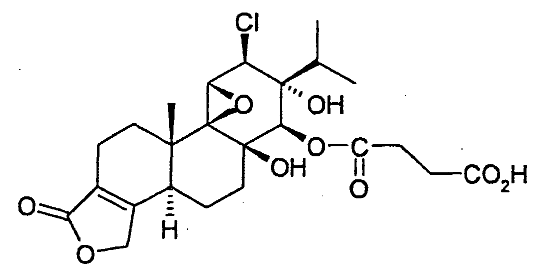
[3bS-(3bα,5aβ,6β,6aβ,7aβ,7bα,8aR*,8bβ)]-mono[3,3b,4,5,5a,6,6a,7a,7b,8b,9,10-dodecahydro-5a-hydroxy-8b-methyl-6a-(1-methylethyl)-1-oxo-1H-bisoxireno[4b,5:6,7]phenanthro[1,2-c]furan-6-yl]ester, pentanedioic acid 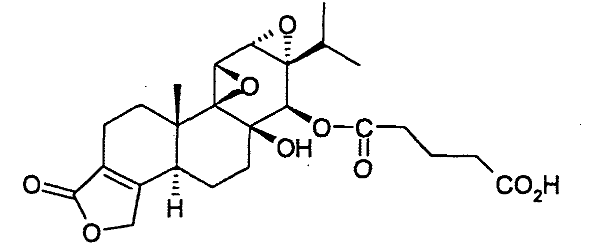
[3bS-(3bα,5aβ,6β,6aβ,7aβ,7bα,8aR*,8bβ,10β)]-3,3b,4,5,5a,6,6a,7a,7b,8b,9,10-dodecahydro-5a-hydroxy-8b-methyl-6a-(1-methylethyl)-1-oxo-1H-bisoxireno[4b,5:6,7]phenanthro[1,2-c]furan-6,10-diyl ester, butanedioic acid 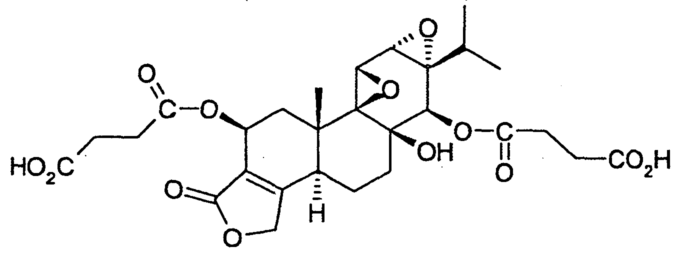
[3bS-(3bα,5aβ,6β,6aβ,7aβ,7bα,8aR*,8bβ)]-mono[2-[6-(3-carboxy-1-oxopropoxy)-1,3,3b,4,5,5a,6,7a,7b,8b,9,10-dodecahydro-5a-hydroxy-8b-methyl-1-oxo-6aH-bisoxireno[4b,5:6,7]phenanthro[1,2-c]furan-6a-yl]-propyl]ester, butanedioic acid 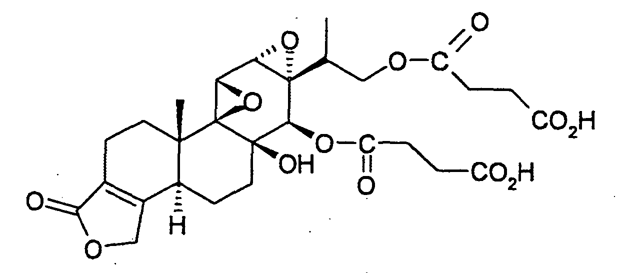
[3bS-(3bα,5aβ,6β,6aβ,7aβ,7bα,8aR*,8bβ)]-3,3b,4,5,5a,6,6a,7a,7b,8b,9,10-dodecahydro-5a-hydroxy-8b-methyl-6a-(1-methylethyl)-1H-bisoxireno[4b,5:6,7]phenanthro[1,2-c]furan-6-yl ester, L-alanine trifluoroacetic acid 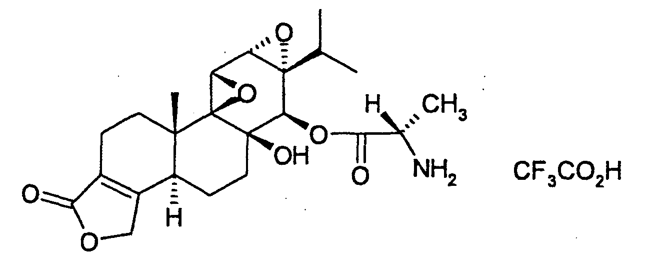
[3bS-(3bα,5aβ,6β,6aβ,7aβ,7bα,8aR*,8bβ)]-3,3b,4,5,5a,6,6a,7a,7b,8b,9,10-dodecahydro-5a-hydroxy-8b-methyl-6a-(1-methylethyl)-1H-bisoxireno[4b,5:6,7]phenanthro[1,2-c]furan-6-yl ester, L-phenylalanine trifluoroacetic acid 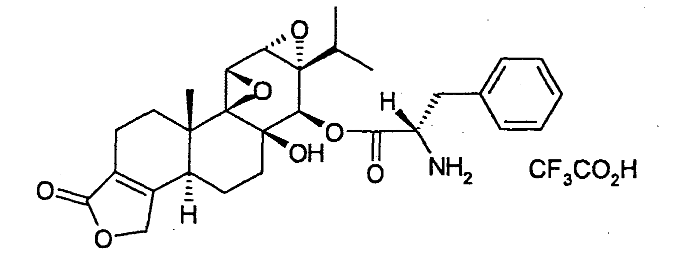
and the like.

WO00/12483 describes triptolide derivatives having the structures shown below.

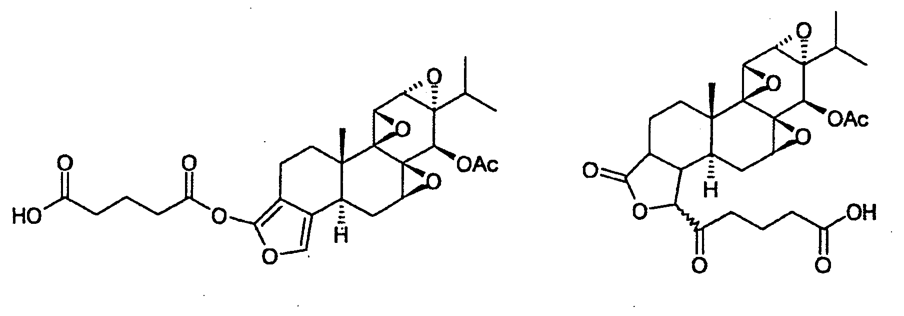

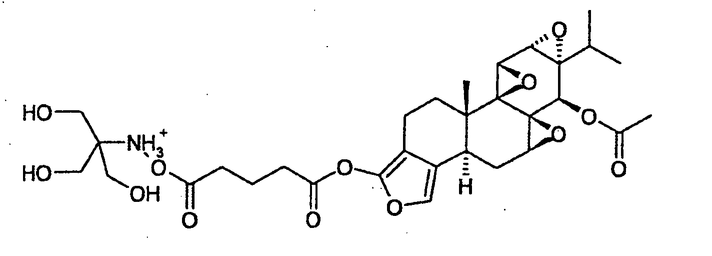

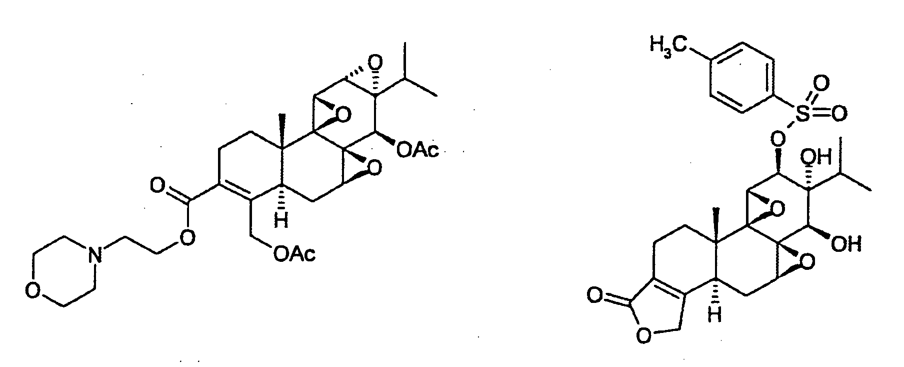

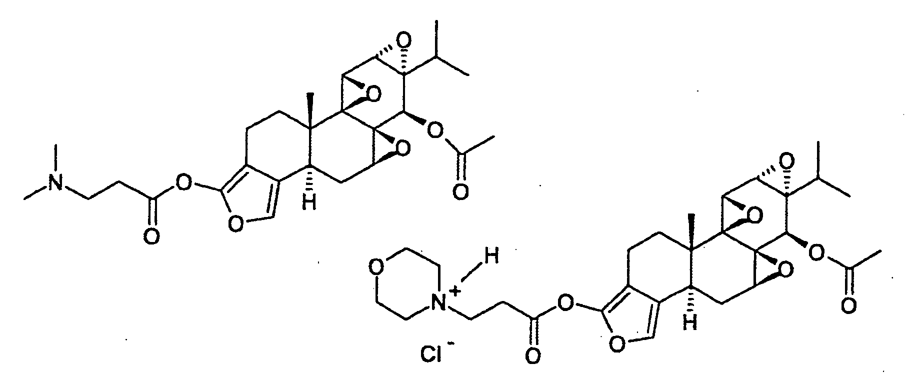

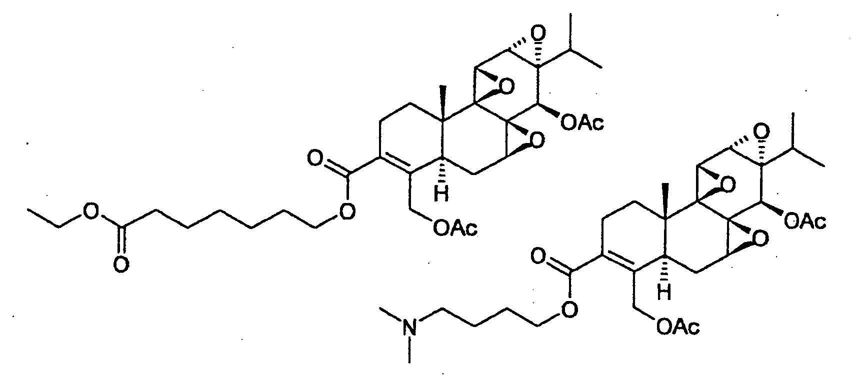

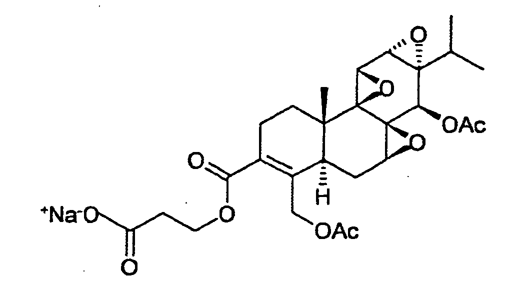

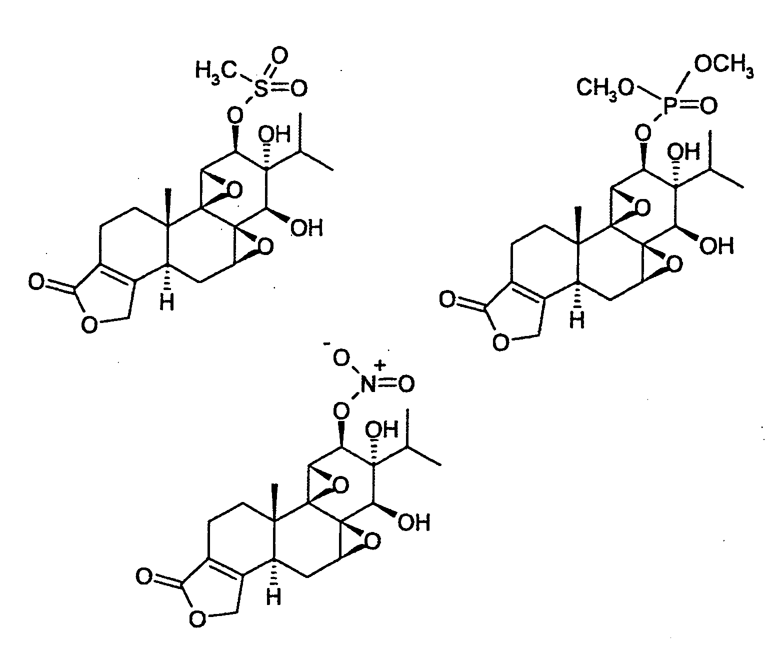

WO02/28862 describes compounds having the structures shown below.

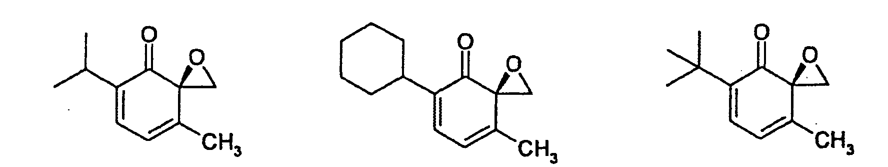

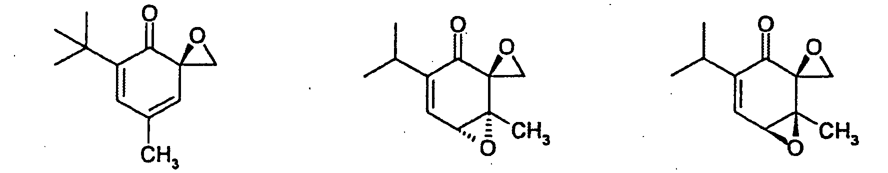

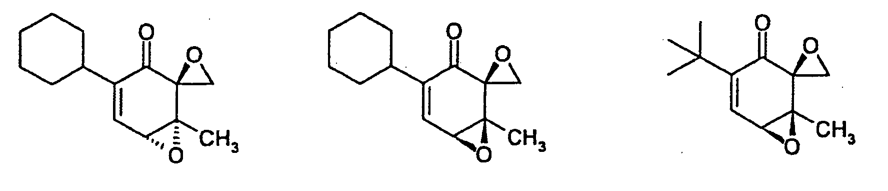

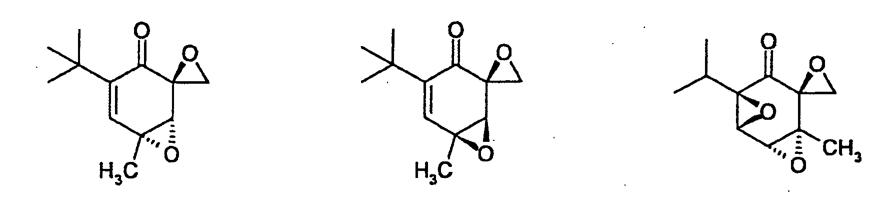

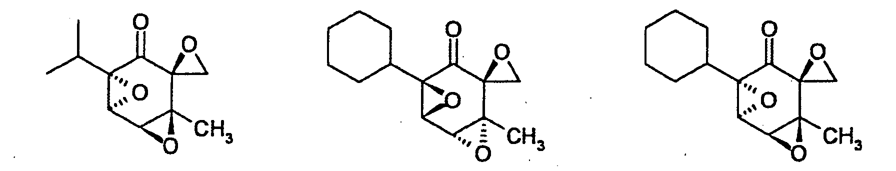

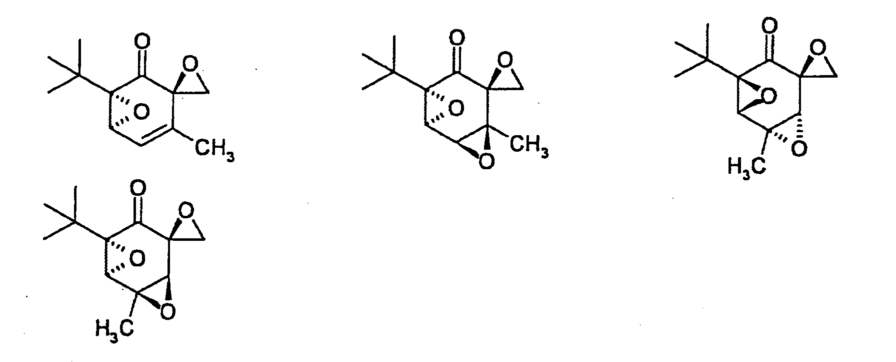

WO00/63212 describes
12-thiocyano-13-hydroxytriptolide

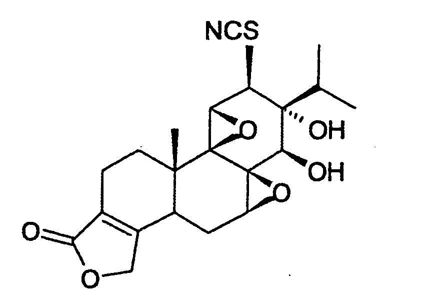

WO98/52933 and US Patent No. 5972998 describe triptolide derivatives such as [5aS-(5aα,5bα,8β,9α,9aR*,10aβ)]-4,5a,5b,8,9,10a,11,11a-octahydro-5b,8,9-trihydroxy-5a-methyl-8-(1-methylethyl)-1H-oxireno[8a,9]phenanthro[1,2-c]furan-3(5H)-one 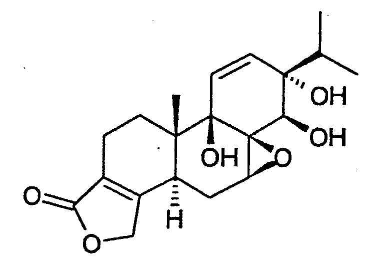
[5aR-(5aα,6α,7β,9aα,9bα)]-3b,4,5,5a,6,7,9a,9b,10,11-decahydro-5a,6,7,9a-tetrahydroxy-9b-methyl-7-(1-methylethyl)-phenanthro[1,2-c]furan-1(3H)-one 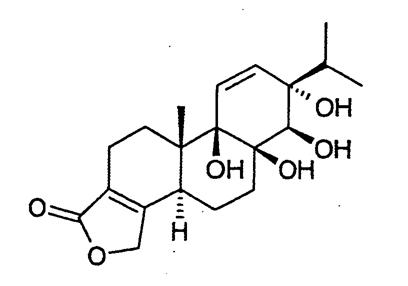
[5R-(5α,5aβ,6β,7α,9aβ,9bβ)]-3b,4,5,5a,6,7,9a,10,11-decahydro-5a,6,9a-trihydroxy-9b-methyl-7-(1-methylethyl)-5,7-epoxyphenanthro[1,2-c]furan-1(3H)-one 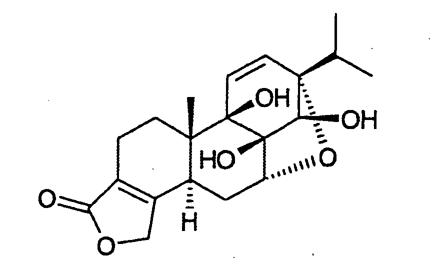
[5aS-(5aα,5bα,8β,9α,9aR*,10aβ)]-4,5a,5b,6,7,8,9,10a,11,11a-decahydro-5b,8,9-trihydroxy-5a-methyl-8-(1-methylethyl)-1H-oxireno[8a,9]phenanthro[1,2-c]furan-3(5H)-one 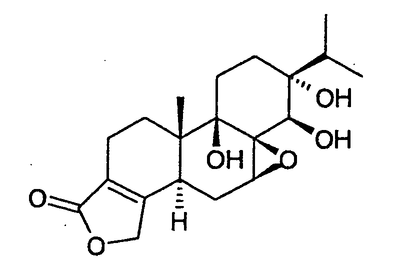
[5aR-(5aα,6α,7β,9aα,9bα)]-3b,4,5,5a,6,7,8,9,9a,9b,10,11-dodecahydro-5a,6,7,9a-tetrahydroxy-9b-methyl-7-(1-methylethyl)-phenanthro[1,2-c]furan-1(3H)-one 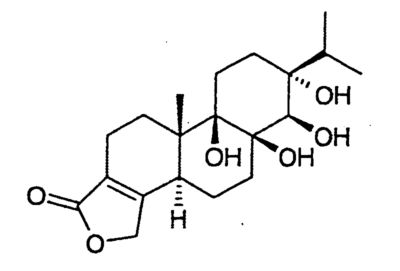
[5R-(5α,5aβ,6pβ7α,9aβ,9bβ)]-3b,4,5,5a,6,7,8,9,9a,9b,10,11-dodecahydro-5a,6,9a-trihydroxy-9b-methyl-7-(1-methylethyl)-5,7-epoxyphenanthro[1,2-c]furan-1(3A)-one 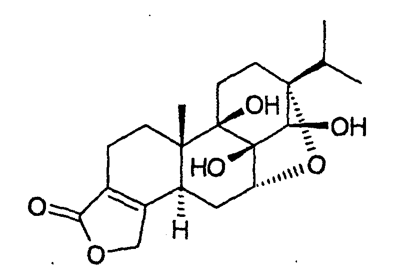
[4S- (4α,5aα,5bα,8β,9α,9aR*,10aβ,11aβ)]-8-(1-methylethyl) - 4,5a,5b,8,9,10a,11,11a-octahydro-4,5b,8,9-tetrahydroxy-5a-methyl-1H-oxireno[8a,9]phenanthro[1,2-c]furan-3(5H)-one 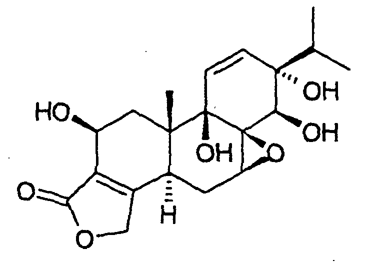
[5aR-(5aα,6α,7β,9aα,9bα,11α)]-3b,9,5,5a,6,7,9a,9b,10,11-decahydro-5a,6,7,9a,11-pentahydroxy-9b-methyl-7-(1-methylethyl)-phenanthro[1,2-c]furan-1(3H)-one 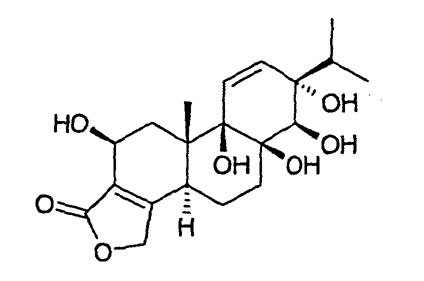
[3bS-(3bα,5α,5aβ,6β,7α,9aβ,9bβ,11β)]-3b,4,5,5a,6,7,9a,9b,10,11-decahydro-5a,6,9a,11-tetrahydroxy-9b-methyl-7-(1-methylethyl)-5,7-epoxyphenanthro[1,2-c]-furan-1(3H)-one 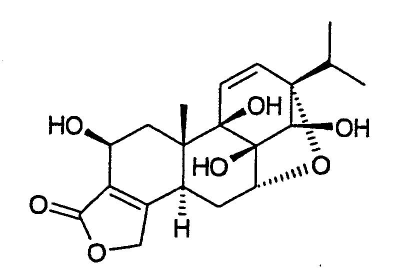
[5aS-(5aα,5bα,8β,9α,9aR*,10aβ,11aβ)]-4,5a,5b,8,9,10a,11,11a-octahydro-5b,8,9-trihydroxy-8-(1-hydroxy-1-methylethyl)-5a-methyl-1H-oxireno[8a,9]phenanthro[1,2-c]furan-3(5H)-one 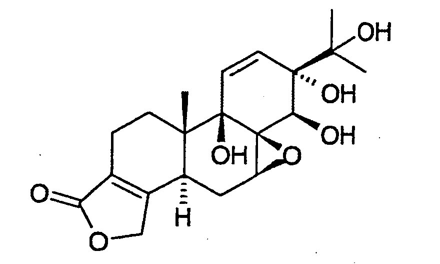
[5aR-(5aα,6α,7β,9aα,9bα)]-3b,4,5,5a,6,7,9a,9b,10,11-decahydro-5a,6,7,9a-tetrahydroxy-7-(1-hydroxy-1-methylethyl)-9b-methyl-phenanthro[1,2-c]furan-1(3H)-one 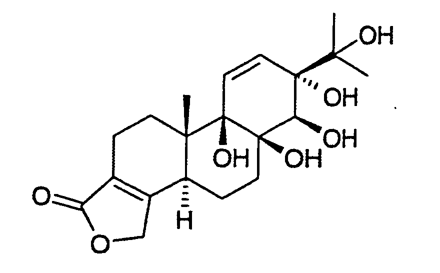
[3bS-(3bα,5α,5aβ,6β,7α,9aβ,9bβ)]-3b,4,5,5a,6,7,9a,9b,10,11-decahydro-5a,6,9a-trihydroxy-7-(1-hydroxy-1-methylethyl)-9b-methyl-5,7-epoxyphenanthro[1,2-c]furan-1(3H)-one 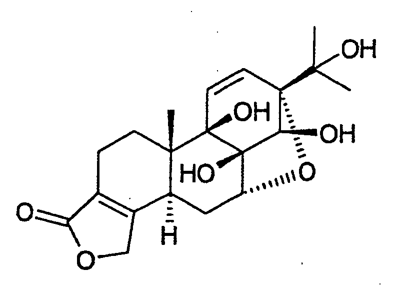
[5aS-(5aα,5bα,8β,9α,9aR*,10aβ)]-4,5a,5b,8,9,10a,11,11a-octahydro-5b,8,9-trihydroxy-5a-methyl-8-(2-hydroxy-1-methylethyl)-1H-oxireno[8a,9]phenanthro[1,2-c]furan-3(5H)-one 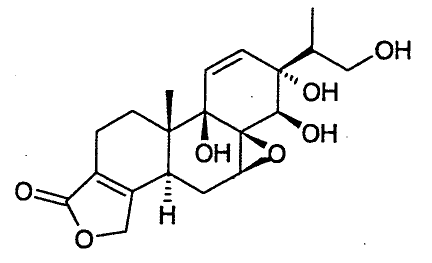
[5aR- (5aα,6α,7β,9aα,9bα)]-3b,4,5,5a,6,7,9a,9b,10,11-decahydro-5a,6,7,9a-tetrahydroxy-7-(2-hydroxy-1-methylethyl)-9b-methyl-phenanthro[1,2-c]furan-1(3H)-one 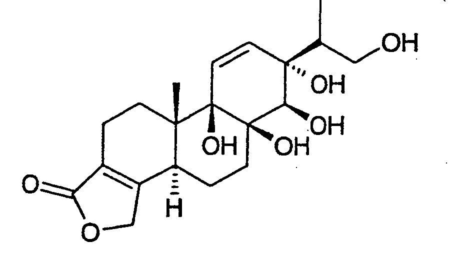
[5R-(5α,5aβ,6β,7α,9aβ,9bβ)]-3b,4,5,5a,6,7,9a,9b,10,11-decahydro-5a,6,9a-trihydroxy-9b-methyl-7-(2-hydroxy-1-methylethyl)-5,7-epoxyphenanthro[1,2-c]furan-1(3H)-one 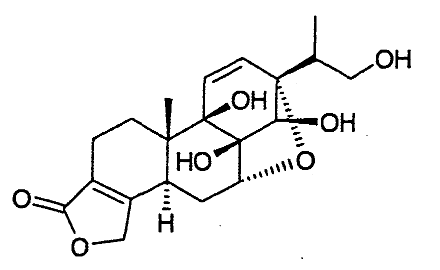
[5aS- (5aα,5bα,8β,9α,9aS*,10aβ,11aβ)]-mono[3,4,5,5a,5b,8,9,10a,11,11a-decahydro-5b,8-dihydroxy-5a-methyl-8-(1-methylethyl)-3-oxo-1H-oxireno[8a,9]phenanthro[1,2-c]furan-9-yl]ester, butanedioic acid 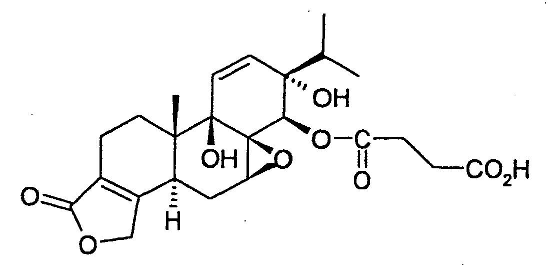
[3bS-(3bα,5aβ,6β,7α,9aβ,9bβ)]-mono[1,3,3b,4,5,5a,6,7,9a,9b,10,11-dodecahydro-5a,7,9a-trihydroxy-9b-methyl-7-(1-methylethyl)-1-oxophenanthro[1,2-c]furan-6-yl]ester, butanedioic acid 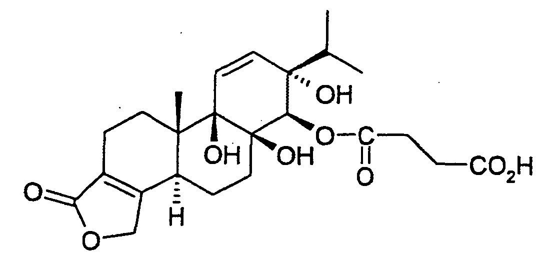
[3bS-(3bα,5α,5aβ,6β,7α,9aβ,9bβ)]-mono[1,3,3b,4,5,5a,6,7,9a,9b,10,11-dodecahydro-5a,9a-dihydroxy-9b-methyl-7-(1-methylethyl)-1-oxo-5,7-epoxyphenanthro[1,2-c]furan-6-yl]ester, butanedioic acid 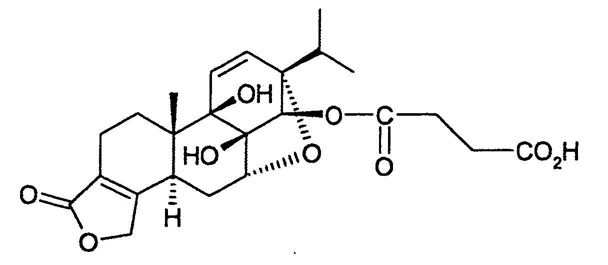
[5aS-(5aα,5bβ,8β,9α,9aS*,10aβ,11aβ)]-mono[3,4,5,5a,8,9,10a,11,11a-decahydro-5b,8-dihydroxy-5a-methyl-8-(1-methylethyl)-3-oxo-1H-oxireno[8a,9]phenanthro[1,2-c]furan-9-yl]ester, pentanedioic acid 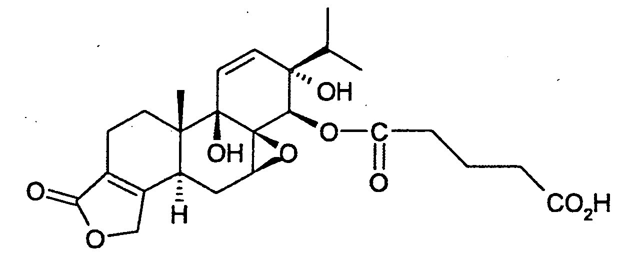
[4S-(4α,5aα,5bα,8β,9α,9aS*,10aβ,11aβ)]-3,4,5,5a,5b,8,9,10a,11,11a-decahydro-5b,8-dihydroxy-5a-methyl-8-(1-methylethyl)-3-oxo-1H-oxireno[8a,9]phenanthro[1,2-c]furan-4,9-diyl ester, butanedioic acid 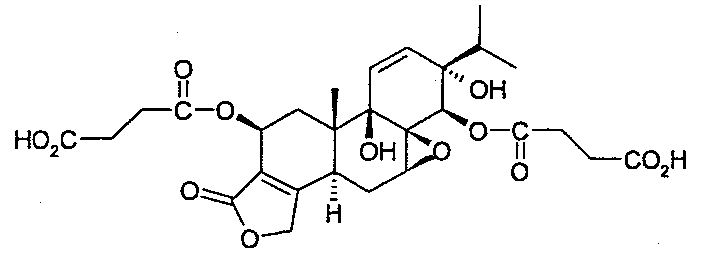
[3bS-(3bα,5aβ,6β,7α,9aβ,9bβ)]-1,3,3b,4,5,5a,6,7,9a,9b,10,11-dodecahydro-5a,7,9a-trihydroxy-9b-methyl-7-(1-methylethyl)-1-oxophenanthro[1,2-c]furan-6-yl ester, L-alanine trifluoroacetic acid 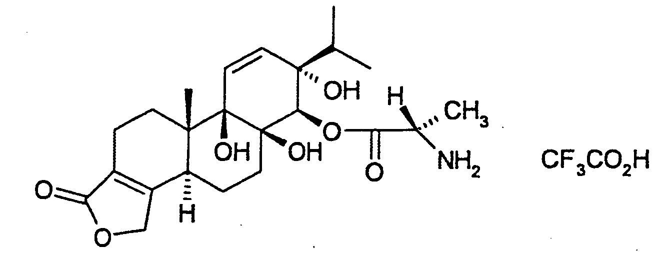
[3bS-(3bα,5aβ,6β,7α,9aβ,9bβ)]-1,3,3b,4,5,5a,6,7,9a,9b,10,11-dodecahydro-5a,7,9a-trihydroxy-9b-methyl-7-(1-methylethyl)-1-oxophenanthro[1,2-c]furan-6-yl ester, L-phenylalanine trifluoroacetic acid 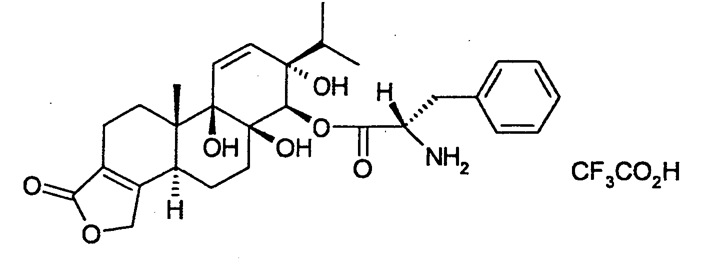
and the like.

WO02/070472 describes triptolide derivatives having the structures shown below.

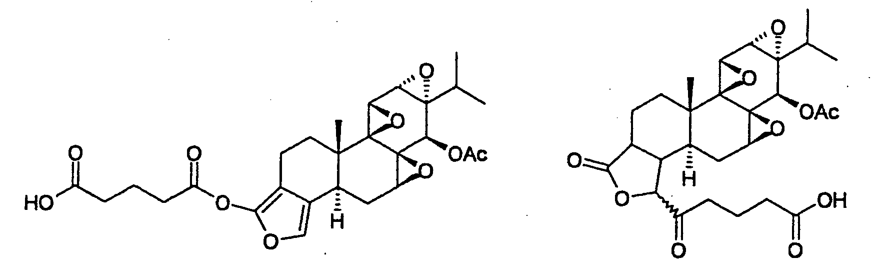

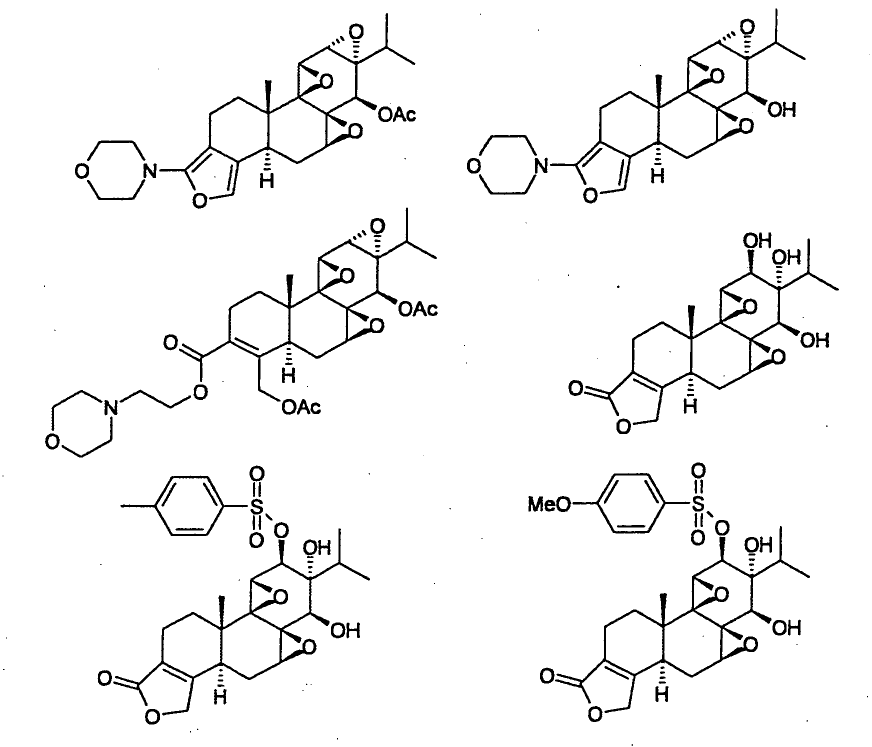

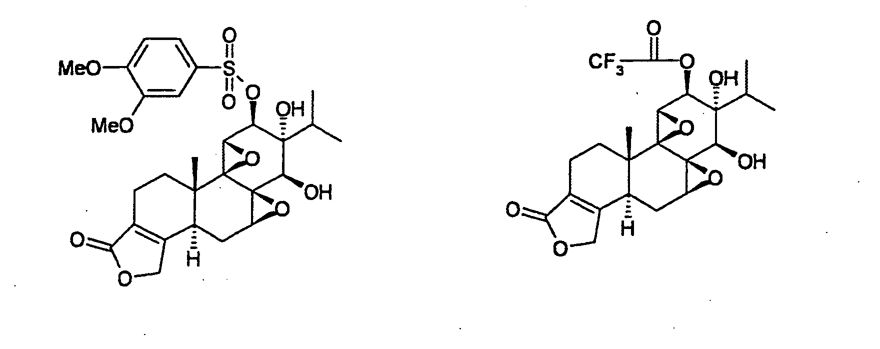

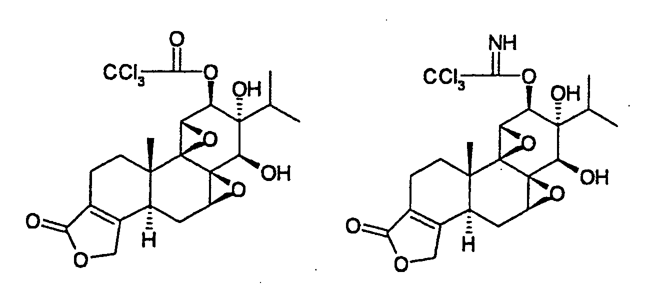

WO02/074759 describes triptolide derivatives such as
triptolide 14-N-tert-butoxycarbonyl-α-t-butyl-L-glutamate ester 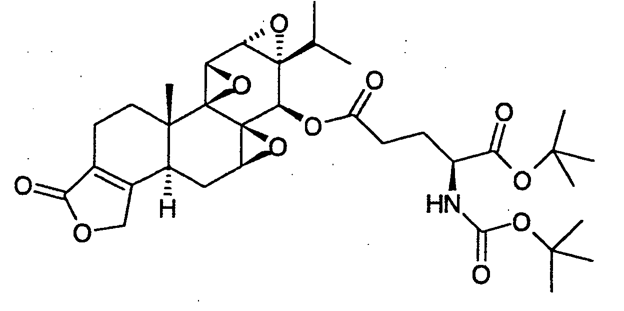
triptolide 14-α-benzyloxycarbonyl-D-glutamate ester 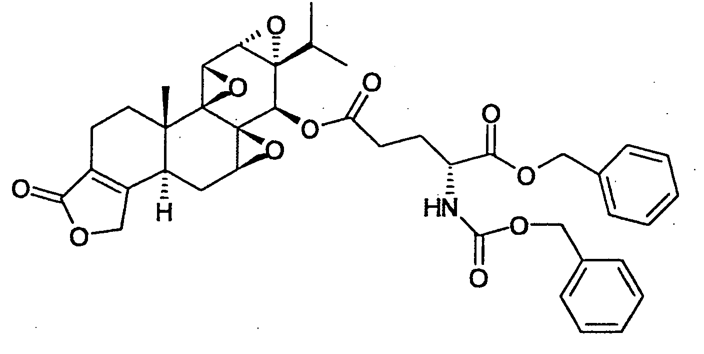
triptolide 14-γ-benzyl-N-benzyloxycarbonyl-(L)-glutamate ester, mixture with D-isomer 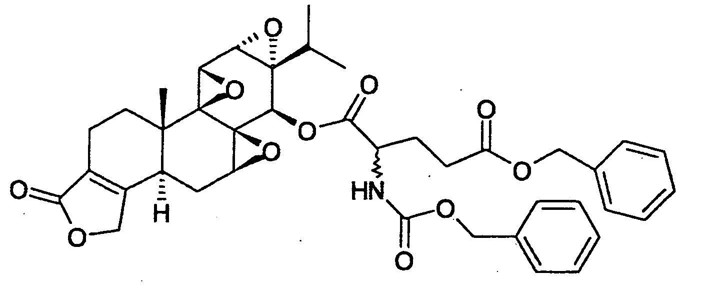
triptolide 14-β-benzyl-N-benzyloxycarbonyl-(L)-aspartate ester, mixture with D-isomer 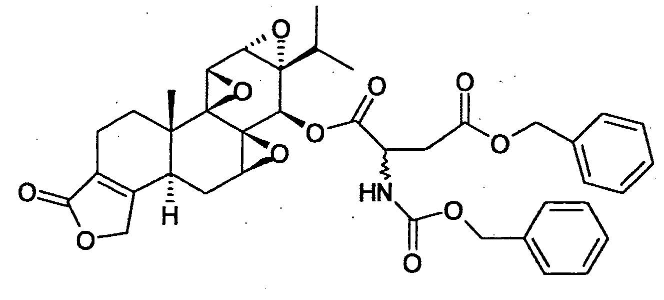
triptolide 14-N-tert-butoxycarbonyl-β-tert-butoxycarbonyl-β-tert-butyl-(L)-aspartate ester, mixture with D-isomer 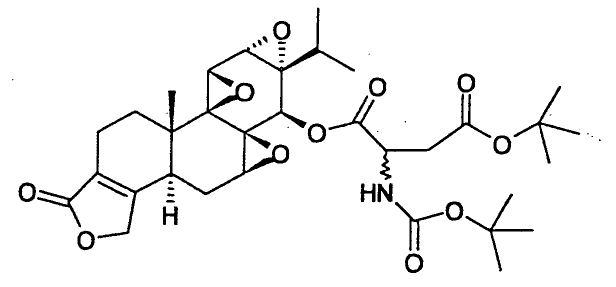
triptolide 14-γ-L-glutamate ester 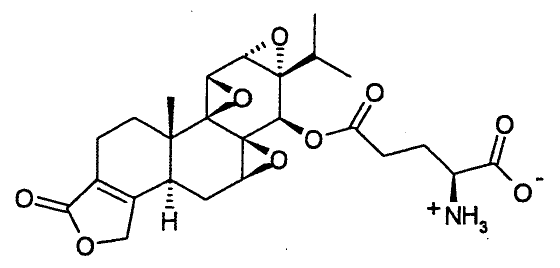
and the like.

Triptolide and a derivative thereof in the form of pharmaceutically acceptable salts can be appropriately used in the present invention. Examples of such salt include acid addition salts, base addition salts and the like including inorganic acid salts such as hydrochloride, hydrobromide, hydroiodide, sulfate, nitrate, phosphate, carbonate, hydrogencarbonate, perchlorate and the like; organic acid salts such as formate, acetate, trifluoroacetate, propionate, oxalate, glycolate, succinate, lactate, maleate, hydroxymaleate, methylmaleate, fumarate, adipate, tartrate, malate, citrate, benzoate, cinnamate, ascorbate, salicylate, 2-acetoxybenzoate, nicotinate, isonicotinate and the like; sulfonates such as methanesulfonate, ethanesulfonate, isothionate, benzenesulfonate, p-toluenesulfonate, naphthalenesulfonate and the like; acidic amino acid salts such as aspartate, glutamate and the like; alkali metal salts such as sodium salt, potassium salt and the like; alkaline earth metal salts such as magnesium salt, calcium salt and the like; ammonium salt; organic base salts such as trimethylamine salt, triethylamine salt, pyridine salt, picbline salt, dicyclohexylamine salt, N,N'-dibenzylethylenediamine salt and the like; basic amino acid salts such as lysine salt, arginine salt and the like can be mentioned. In some cases, hydrate, or solvate with alcohol and the like may be used.

The therapeutic agent for corneal ulcer of the present invention can contain any component besides triptolide and a derivative thereof as long as it does not adversely affect the corneal ulcer treating action of triptolide and a derivative thereof. As such component, for example, antimicrobial agent, immunosuppressant, anti-inflammatory agent, anti-allergic drug, antiviral agent, vasoconstrictor and the like can be mentioned. Specifically, antimicrobial agents such as lomefloxacin hydrochloride, ofloxacin, norfloxacin, levofloxacin, erythromycin, sulfisoxazol, oxytetracycline hydrochloride, polyoxin B sulfate, cefmenoxime hydrochloride, chloramphenicol, sulbenicillin, tobramicin, gentamicin sulfate, sisomicin sulfate, fradiomycin sulfate, micromicin sulfate and the like; immunosuppressants such as cyclosporin, rapamicin, tacrolimus and the like; anti-inflammatory agents such as azulene, indomethacin, lysozyme hydrochloride, dichlofenak sodium, pranoprofen and the like; anti-allergic agents such as ketotifen fumarate, tranilast, metaquizine, diphenhydramine and the like; antiviral agents such as acyclovir, idoxuridine, pimaricin and the like; vasoconstrictors such as tetrahydrozoline nitrate, oxymetazoline hydrochloride and the like; and the like can be mentioned.

The therapeutic agent for corneal ulcer of the present invention may contain additives generally used in the pertinent field as appropriate according to the administration form.

While the administration route of the therapeutic agent for corneal ulcer of the present invention is not particularly limited as long as it shows a treatment effect of corneal ulcer, topical administration to the eye is preferable. As the dosage form of ophthalmic topical administration, for example, ophthalmic solutions and ophthalmic ointments can be mentioned.

For example, when the therapeutic agent for corneal ulcer of the present invention is used as an ophthalmic solution or an ophthalmic ointment, stabilizers (e.g., sodium bisulfite, sodium thiosulfate, sodium edetate, sodium citrate, ascorbic acid, dibutylhydroxytoluene and the like), auxiliary solvents (e.g., glycerol, propyleneglycol, macrogol, polyoxyethylene hydrogenated castor oil and the like), suspending agents (e.g., polyvinylpyrrolidone, hydroxypropylmethylcellulose, hydroxymethylcellulose, sodium carboxymethylcellulose and the like), emulsifiers (e.g., polyvinylpyrrolidone, soybean lecithin, egg yolk lecithin, polyoxyethylene hydrogenated castor oil, polysorbate80 and the like), buffers (e.g., phosphate buffer, acetate buffer, borate buffer, carbonate buffer, citrate buffer, tris buffer, glutamic acid, ε-aminocaproic acid and the like), viscous agents (e.g., water-soluble cellulose derivatives such as methylcellulose, hydroxyethylcellulose, hydroxypropylmethylcellulose, carboxymethylcellulose and the like, sodium chondroitin sulfate, sodium hyaluronate, carboxyvinyl polymer, polyvinyl alcohol, polyvinyl pyrrolidone, macrogol and the like), preservatives (e.g., benzalkonium chloride, benzethonium chloride, chlorhexidine gluconate, chlorobutanol, benzyl alcohol, sodium dehydroacetate, paraoxybenzoates, sodium edetate, boric acid and the like), isotonic agents (e.g., sodium chloride, potassium chloride, glycerol, mannitol, sorbitol, boric acid, glucose, propylene glycol and the like), pH adjusting agents (e.g., hydrochloric acid, sodium hydroxide, phosphoric acid, acetic acid and the like), refreshments (e.g., 1-menthol, d-campher, d-borneol, peppermint oil and the like), ointment bases (white petrolatum, purified lanolin, liquid paraffin, vegetable oils (olive oil, camellia oil, peanut oil and the like) and the like) and the like can be added. While the amount of these additives varies depending on the kind, use and the like of the additives, the additives may be added at a concentration sufficient to achieve the object.

When the therapeutic agent for corneal ulcer of the present invention is processed to give an ophthalmic solution or an ophthalmic ointment, it can be produced according to the method generally employed in the field of formulation of preparations. For example, it can be produced according to the method described in the Japanese Pharmacopoeia, Fourteenth Edition, General Rules of Preparations, the section of Ophthalmic Solution and the section of Ophthalmic Ointment.

The subject of the administration of the therapeutic agent for corneal ulcer of the present invention is not particularly limited, and the therapeutic agent is useful for the corneal ulcer treatment in various mammals including human, monkey, mouse, rat, rabbit, pig, dog, horse, bovine and the like.

When the therapeutic agent for corneal ulcer of the present invention is administered to human, a solution containing triptolide or a derivative thereof at a concentration of about 100 nM - about 1000 µM, preferably about 1 µM - about 100 µM, needs only to be administered once to 6 times a day by 1-2 drops to one eye per administration.

### Examples

The present invention is explained in more detail by referring to Examples, which are not to be construed as limitative.

### Example 1. Triptolide

### Preparation of reagents

### [Preparation of triptolide solution]

Triptolide[PG490] (1 mg, manufactured by ALEXIS BIOCHEMICALS, Switzerland, isolated from *Tripterygium wilfordii,* purity 98%) was dissolved in dimethyl sulfoxide (DMSO) (0.925 ml) (3.0×10⁻³ M). The solution was diluted 500-fold (6.0×10⁻⁶ M) with serum free Eagle's minimum essential medium (MEM), and thereafter serially 10-fold diluted with serum free MEM containing 0.2% DMSO.

### [Preparation of dexamethasone solution]

Dexamethasone (39.25 mg, manufactured by Sigma Aldrich Japan) was dissolved in DMSO (1.0 ml) (1.0×10⁻¹ M). The solution was diluted 500-fold (2.0×10⁻⁴ M) with serum free MEM, and thereafter serially 10-fold diluted with serum free MEM containing 0.2% DMSO.

### [Preparation of Ehrlich's reagent solution]

60% Perchloric acid (10 ml) and 2-propanol (65 ml) were mixed and dissolved by adding p-dimethylaminobenzaldehyde (6.6 g).

### [Preparation of hydroxyproline standard solution]

Hydroxyproline (20 mg) was dissolved in the total amount (50 ml) of distilled water to give 400 µg/ml of hydroxyproline solution, which was preserved in a refrigerator.

20 µg/ml, 15 µg/ml, 10 µg/ml, 5 µg/ml and 0 µg/ml HYP solutions were prepared from 400 µg/ml hydroxyproline (HYP) solution.

### [Animals used]

Japanese albino rabbits (body weight 2.5-3.0 kg, male) were purchased from Biotech (Japan) and used.

### [Other substances used]

MEM and tissue culture medium 199 (TCM199) used were those manufactured by Gibco (U.S.A.). The tissue culture plate used was one manufactured by Corning Costar Corporation (U.S.A.). Ultra-free (trademark)-MC filter was one manufactured by Millipore Corporation (U.S.A.). Native porcine Type I collagen (acid solubilized) and 5-fold concentration of Dulbecco's modified Eagle minimum essential medium (DMEM) were obtained from Nitta Gelatin (Japan). Bovine plasminogen was obtained from Sigma (U.S.A.). IL-1β was obtained from R&D Systems (U.S.A.). S-2251 (H-D-Val-Leu-Lys-pNA) was obtained from Daiichi Pure Chemicals Co., Ltd. (Japan). Sheep anti-rabbit MMP-1 antibody and sheep anti-rabbit MMP-3 antibody used were provided by Mr. Nagase of Department of Biochemistry and Molecular Biology, University of Kansas Medical Center. Preparation of corneal stromal cell

Rabbit corneal stromal cells were prepared and cultured according to the description of Mishima et al., Invest. Ophthalmol. Vis. Sci. 28, 1521-6 (1987). The rabbits were sacrificed by overdose of sodium pentobarbital. Eyeballs were enucleated, sclera was incised to remove cornea. Stroma was separated from epithelium and endothelium. Corneal stromal cells were detached from stroma by digesting with collagenase (Collagenase from Clostridium histolyticum, Sigma (U.S.A.) #C-0130) (2 mg/ml) at 37°C for 3-4 hr. The cells were cultured in MEM supplemented with 10% fetal bovine serum (FBS), grown in an incubator (37°C humidity 100%) in 5% CO₂ and then passaged in MEM containing 10% FBS.

### Three-dimensional culture

Collagen gel was prepared according to the manual of Nitta gelatin. To be specific, the obtained rabbit corneal stromal cells were washed with PBS(-), 0.05% Trypsin (1 ml) was added thereto, and the mixture was left in a CO₂ incubator for several min to detach the cells. MEM (5 ml) containing 10% FBS was added to quench the trypsin reaction. The cell suspension was transferred to a 15 ml centrifuge tube, and centrifuged at 1500 rpm for 5 min. The supernatant was removed and the cells were suspended in a constant amount of serum free MEM. The number of the cells was counted with Burker-Turk hemocytometer (Kayagaki Irika Kogyo). The supernatant was removed by centrifugation at 1500 rpm for 5 min. Serum free MEM was added to give a cell suspension (2200000 cells/ml). A. acid-solubilized Type I collagen (3 mg/ml), B. 5-fold concentration of DMEM, C. reconstruction buffer (0.05N NaOH, 0.26 M NaHCO₃, 0.2 M HEPES, pH 7.3) and D. a cell suspension (2.2×10⁶ cells/ml) were mixed at a ratio of A:B:C:D=7:2:1:1 while cooling on ice. The collagen solution was placed in a 24 well culture plate at 0.5 ml/well, and stood in an incubator at 37°C 5% CO₂ for 0.5-5 hr. Then, 0.5 ml of serum free MEM containing IL-1β (0.1 ng/mL) and various concentrations of triptolide or dexamethasone was overlaid and cultured for 48 hr.

### Experimental Example

### [Measurement of collagen degradation activity]

After three-dimensional culture, the medium was collected and non-degraded collagen fibril having a molecular weight exceeding 100 kDa was removed by ultrafiltration. The filtrate was hydrolyzed by heat block at 110°C for 24 hr using hydrochloric acid. The amount of the hydroxyproline in the hydrolysate was measured using an Ehrlich's reagent and a spectrophotometer (Bergman et al., Anal. Chem., 35, 1961-5 (1963)). The amount of the degraded collagen was expressed by the amount of hydroxyproline per well.

### [Western blot]

After three-dimensional culture, the collected medium was electrophoresed on 12.5% SDS/polyacrylamide gel under reduction conditions, and the separated protein was transferred onto a PVDF membrane (Immobilon (trademark)-P, manufactured by Millipore, U.S.A.). The transferred membrane was blocked and reacted with sheep anti(rabbit MMP-1)antibody or sheep anti(rabbit MMP-3)antibody. An immunodetection of the antigen was performed using an ECL (trademark) Western blot detection kit (manufactured by Amersham, UK). Each MMP was identified based on the reactivity of the antibody and molecular weight.

### [Gelatin zymography]

After three-dimensional culture, the collected medium was separated using 10% SDS/polyacrylamide gel containing 0.1% gelatin according to the method described in Birkedal-Hansen et al., Biochem. Biophys. Res. Commun. 107, 1173-8 (1982). The sample medium was mixed with an SDS-sample buffer (125 mM Tris, pH 6.8, 20% glycerol, 2% SDS, 0.002% bromophenol blue) free of a reducing agent. After electrophoresis at 4°C, the gel was washed with 2.5% Triton X-100 for 1 hr to allow recovery of the protease activity. Then, the gel was incubated in a reaction buffer (50 mM Tris, pH 7.5, 5 mM CaCl₂ and 1% Triton X-100) at 37°C for 18 hr. The gel was stained with Coomassie Brilliant Blue and each MMP was identified based on the molecular weight.

### [RT-PCR]

After three-dimensional culture, collagenase was added to dissolve the collagen gel, and the cells were collected. The total RNA was extracted from the cells using RNeasy Mini Kit (manufactured by Qiagen, Germany), and the total RNA (1 µg) was converted to cDNA using Reverse Transcription System (manufactured by Promega, U.S.A.). This cDNA, QuantiTect SYBR Green PCR Master Mix solution (QuantiTect-SYBR Green PCR kit, manufactured by Qiagen, Germany), nuclease free water and rabbit TIMP-1 primer (synthesis at Nippon Gene Institute, Han et al., Nagoya J. Med. Sci. 62, 115-126 (1999)) were mixed, and the expression amount of TIMP-1 mRNA was examined using LightCycler (manufactured by Roche Molecular Biochemicals, Germany).

### Experimental Example 1

The effect of triptolide on IL-1β induced collagen degradation was examined. After the above-mentioned three-dimensional culture using a triptolide solution, an experiment was conducted according to the method described in the above-mentioned measurement of collagenolytic activity. The results are shown in Fig. 1.

According to Fig. 1, it is clear that triptolide suppresses IL-1β induced collagen degradation in a concentration dependent manner.

### Experimental Example 2

The effect of triptolide on the expression of MMP and proMMPs in rabbit corneal stromal cells was examined. After the above-mentioned three-dimensional culture using a triptolide solution, an experiment was conducted according to the method described in the above-mentioned Western blot or gelatin zymography. The results are shown in Fig. 2.

According to Fig. 2, it is clear that triptolide suppresses increase of glycosylated proMMP-1, proMMP-1 and proMMP-3 induced by IL-1β.

### Experimental Example 3

The effect of triptolide on the expression of MMPs and proMMPs in rabbit corneal stromal cells was examined. After the above-mentioned three-dimensional culture using a triptolide solution, an experiment was conducted according to the method described in the above-mentioned Western blot or gelatin zymography. The results are shown in Fig. 3.

According to Fig. 3, it is clear that triptolide suppresses increase and activation of glycosylated proMMP-1, proMMP-1 and proMMP-3, as well as activation of proMMP-9, which are induced by IL-1β and plasminogen.

### Experimental Example 4

The effect of dexamethasone on IL-1β induced collagen degradation was examined. After the above-mentioned three-dimensional culture using a dexamethasone solution, an experiment was conducted according to the method described in the above-mentioned measurement of collagenolytic activity. The results are shown in Fig. 4.

According to Fig. 4, it is clear that dexamethasone suppresses IL-1β induced collagen degradation in a concentration dependent manner.

### Experimental Example 5

The effect of dexamethasone on the expression of MMP and proMMPs in rabbit corneal stromal cells was examined. After the above-mentioned three-dimensional culture using a dexamethasone solution, an experiment was conducted according to the method described in the above-mentioned Western blot or gelatin zymography. The results are shown in Fig. 5.

According to Fig. 5, it is clear that dexamethasone suppresses increase of glycosylated proMMP-1, proMMP-1 and proMMP-3 induced by IL-1β.

### Experimental Example 6

The effect of triptolide on the expression of MMPs and proMMPs in rabbit corneal stromal cells was examined. After the above-mentioned three-dimensional culture using a triptolide solution, an experiment was conducted according to the method described in the above-mentioned Western blot or gelatin zymography. The results are shown in Fig. 6.

According to Fig. 6, it is clear that dexamethasone suppresses activation of proMMP-1, increase of proMMP-3, as well as activation of proMMP-2 and proMMP-9, which are induced by IL-1β and plasminogen.

### Experimental Example 7

The effect of triptolide and dexamethasone on TIMP-1 production was examined. An experiment was conducted according to the method described in the above-mentioned RT-PCR. The results are shown in Fig. 7.

According to Fig. 7, it is clear that dexamethasone suppresses TIMP-1 production (mRNA level) induced by IL-1β, but triptolide does not.

The suppression of TIMP-1 production by dexamethasone means suppression of expression amount of endogenous MMP inhibitor, potentially promoting collagenolysis. However, since triptolide does not affect TIMP-1 production, triptolide is considered to more effectively suppress collagen degradation than does dexamethasone.

### Example 2. triptolide succinate sodium salt

### triptolide succinate

Triptolide[PG490] (manufactured by CALBIOCHEM, U.S.A., isolated from *Tripterygium wilfordii,* purity 98%) (0.05 g, 0.13 mmol) and 4-dimethylaminopyridine (0.017 g, 0.14 mmol) were dissolved in a mixed solution of dimethylformamide (1 mL) and pyridine (5 mL) and succinic anhydride (0.075 g, 0.75 mmol) was added. The mixture was stirred under a nitrogen atmosphere at 85°C for 30 min. The solvent was evaporated under reduced pressure and 1N aqueous potassium hydroxide solution (2 mL) was added. The mixture was washed with ethyl acetate. The aqueous layer was acidified with 1N hydrochloric acid and extracted with ethyl acetate (100 mL). The organic layer was washed with saturated brine and dried over anhydrous sodium sulfate. The solvent was evaporated under reduced pressure to give triptolide succinate (0.012 g, 18.7%) as a yellow solid. ¹H-NMR spectrum (300MHz, CDCl₃)δ: 0.83 (3H, d, J=6.6Hz), 0.94 (3H, d, J=7.2Hz), 1.05(3H, s), 1.21(1H, m), 1.55(1H, m), 1.89(2H, m), 2.15(2H, m), 2.30(1H, m), 2.74-2.76(5H, m), 3.45(1H, d, J=5.7Hz), 3.52(1H, d, J=3.3Hz), 3.82(1H, d, J=3.0Hz), 4.66(2H, s), 5.09(1H, s).

### triptolide succinate sodium salt

Water (20 mL) was added to triptolide succinate (0.01 g, 0.02 mmol) obtained above and sodium hydrogen carbonate (0.002 g, 0.02 mmol) and the mixture was stirred at room temperature for 30 min. The aqueous solution was filtered, and the filtrate was lyophilized to give a triptolide succinate sodium salt (0.01 g, 95.4%) as a yellow foam.
¹H-NMR spectrum (300MHz, DMSO-d₆)δ: 0.74 (3H, d, J=7.2Hz), 0.86(3H, d, J=6.9Hz), 0.91 (3H, s, 20-CH₃), 1.28-1.30 (2H, br), 1.80-2.23(7H, m), 2.38-2.51 (3H, m), 3.55(1H, m), 3.70(1H, m), 3.95(1H, d, J=3.0Hz), 4.83(2H, brs), 4.97(1H, s, 14-CH).

### Preparation of reagent

As test substances, triptolide[PG490] (manufactured by CALBIOCHEM, U.S.A., isolated from *Tripterygium wilfordii*, purity 98%) and triptolide succinate sodium salt obtained above were used.

### [Preparation of triptolide solution]

Triptolide[PG490] (1 mg, manufactured by CALBIOCHEM, U.S.A., isolated from *Tripterygium wilfordii,* purity 98%) was dissolved in DMSO (27.7 µL) (1.0×10⁻¹ M). This was diluted 10-fold (1.0×10⁻² M) with DMSO. Each solution prepared using DMSO was 500-fold diluted (2.0×10⁻⁴ M, 2.0×10⁻⁵ M) with serum free MEM.

### [Preparation of triptolide succinate sodium salt solution]

Triptolide succinate sodium salt (2.17 mg) obtained above was dissolved in DMSO (45 µL) (1.0×10⁻¹ M) and 10-fold diluted (1.0×10⁻² M) with DMSO. Each solution prepared using DMSO was 500-fold diluted (2.0×10⁻⁴ M, 2.0×10⁻⁵ M) with serum free MEM.

### [animals used]

Japanese albino rabbits (body weight about 2 kg, male, purchased from KITAYAMA LABES Co., Ltd.) were used.

As for others, the same procedures as in Example 1 were performed.

### Experimental Example 8

The effect of triptolide and triptolide succinate sodium salt on IL-1β induced collagen degradation of rabbit corneal stromal cells was examined. After the above-mentioned three-dimensional culture using a test substance solution, an experiment was conducted according to the method described in the above-mentioned measurement of collagenolytic activity. The results are shown in Fig. 8.

According to Fig. 8, it is clear that triptolide and triptolide succinate sodium salt suppress IL-1β induced collagen degradation in a concentration dependent manner.

### Experimental Example 9

The effect of triptolide and triptolide succinate sodium salt on the expression of MMPs and proMMPs in rabbit corneal stromal cells was examined. After the above-mentioned three-dimensional culture using a test substance solution, an experiment was conducted according to the method described in the above-mentioned gelatin zymography. The results are shown in Fig. 9.

According to Fig. 9, it is clear that triptolide suppresses increase of proMMP-2 and MMP-2, as well as activation of proMMP-9, which are induced by IL-1β, and triptolide succinate sodium salt suppresses increase of MMP-2 and activation of MMP-9.

| **Formulation Example 1:** ophthalmic solution | |
|---|---|
| triptolide | 3.6 mg |
| polysorbate 80 | 0.1 g |
| sodium dihydrogenphosphate | 0.1 g |
| sodium chloride | 0.9 g |
| benzalkonium chloride | 0.005 g |
| sodium hydroxide | suitable amount (pH 7.0) |
| sterilized purified water | total 100 mL |

The above are admixed to give an ophthalmic suspension.

| **Formulation Example 2:** ophthalmic solution | |
|---|---|
| triptolide | 36 mg |
| boric acid | 700 mg |
| borax | suitable amount |
| sodium chloride | 500 mg |
| hydroxymethylcellulose | 500 mg |
| sodium edetate | 0.05 mg |
| benzalkonium chloride | 0.0005 mg |
| sterilized purified water | total 100 mL |

The above are admixed to give an ophthalmic suspension.

| **Formulation Example 3:** ophthalmic ointment | |
|---|---|
| triptolide | 3.6 mg |
| purified lanolin | 10 g |
| white petrolatum | 100 g |

The above are admixed to give an ophthalmic ointment.

| **Formulation Example 4:** ophthalmic ointment | |
|---|---|
| triptolide | 3.6 mg |
| soybean oil | 5 g |
| white petrolatum | 100 g |

The above are admixed to give an ophthalmic ointment.

| **Formulation Example 5:** ophthalmic solution | |
|---|---|
| triptolide succinate sodium salt | 4.8 mg |
| boric acid | 700 mg |
| borax | suitable amount |
| sodium chloride | 500 mg |
| hydroxymethylcellulose | 500 mg |
| sodium edetate | 0.005 mg |
| benzalkonium chloride | 0.0005 mg |
| sterilized purified water | total 100 mL |

The above are admixed to give an ophthalmic suspension.

| **Formulation Example 6:** ophthalmic ointment | |
|---|---|
| triptolide succinate sodium salt | 4.8 mg |
| purified lanolin | 10 g |
| white petrolatum | 100 g |

The above are admixed to give an ophthalmic ointment.

### Industrial Applicability

According to the present invention, corneal ulcer can be treated without the fear of side effects such as those with steroid.

This application is based on application No. 2003-052072 filed in Japan, the contents of which are incorporated hereinto by reference.

## Claims

1. A therapeutic agent for corneal ulcer, which comprises triptolide or a derivative thereof or a pharmaceutically acceptable salt thereof.

2. The therapeutic agent of claim 1, which is used for topical administration to the eye.

3. The therapeutic agent of claim 2, which is an ophthalmic solution.

4. A pharmaceutical composition for the treatment of corneal ulcer, which comprises triptolide or a derivative thereof or a pharmaceutically acceptable salt thereof.

5. The pharmaceutical composition of claim 4, which is used for topical administration to the eye.

6. The pharmaceutical composition of claim 5, which is used for an ophthalmic solution.

7. A method for treating corneal ulcer, which comprises administering triptolide or a derivative thereof or a pharmaceutically acceptable salt thereof.

8. The treatment method of claim 7, wherein the administration is a topical administration to the eye.

9. The treatment method of claim 8, wherein the topical administration is that of an ophthalmic solution.

10. Use of triptolide or a derivative thereof or a pharmaceutically acceptable salt thereof, for the production of a therapeutic agent for corneal ulcer.

11. The use of claim 10, wherein the therapeutic agent for corneal ulcer is used for topical administration to the eye.

12. The use of claim 11, wherein the therapeutic agent for corneal ulcer is an ophthalmic solution.

13. A commercial package comprising the therapeutic agent of any of claims 1 to 3 and a written matter associated therewith, the written matter stating that the therapeutic agent can or should be used for treating corneal ulcer.

14. A commercial package comprising the pharmaceutical composition of any of claims 4 to 6 and a written matter associated therewith, the written matter stating that the pharmaceutical composition can or should be used for treating corneal ulcer.
